# EUROPÄISCHE PATENTSCHRIFT

(11) **EP 1 145 734 B1**
(45) Veröffentlichungstag und Bekanntmachung des Hinweises auf die Patenterteilung: **23.11.2005**
(21) Anmeldenummer: 01109192.3
(22) Anmeldetag: 12.04.2001
(51) Int. Cl.: A61N 1/36

(54) **Mindestens teilimplantierbares System zur Rehabilitation einer Hörstörung**
At least partially implantable system for the rehabilitation of a hearing disorder
Système au moins partiellement implantable de réhabilitation d'un trouble auditif

(30) Priorität: 13.04.2000 DE 10018334
(43) Veröffentlichungstag der Anmeldung: 17.10.2001
(73) Patentinhaber: Cochlear Limited, Lane Cove, NSW 2066 (AU)
(72) Erfinder: Leysieffer, Hans, Dr.-Ing., 82024 Taufkirchen (DE)
(74) Vertreter: Schwan - Schwan - Schorer

(56) Entgegenhaltungen:
- WO-A-01/56521
- WO-A-99/08480
- US-A- 4 850 962
- US-A- 4 923 469
- US-A- 5 411 467
- US-A- 5 935 166
- R. LASZIG: "Cochlear Implant bei Resthörigkeit" HNO, Bd. 10, 1997, Seiten 740-741, XP002265333

## Beschreibung

Die vorliegende Erfindung betrifft ein mindestens teilweise implantierbares System zur Rehabilitation einer Hörstörung, das mindestens einem Sensor (Mikrofon) zur Aufnahme des Schallsignals und dessen Umwandlung in entsprechende elektrische Signale, eine elektronische Signalverarbeitungseinheit zur Audiosignalverarbeitung und -verstärkung, eine elektrische Energieversorgungseinheit, die einzelne Komponenten des Systems mit Strom versorgt, sowie eine ausgangsseitige aktorische Stimulationsanordnung umfasst.

Unter dem Begriff "Hörstörung" sollen vorliegend Innenohrschäden, kombinierte Innen- und Mittelohrschäden sowie auch zeitweise auftretende oder permanente Ohrgeräusche (Tinnitus) verstanden werden.

Die Rehabilitation sensorischer Hörstörungen mit teilimplantierbaren, elektronischen Systemen hat in den letzten Jahren einen bedeutenden Stellenwert erhalten. Insbesondere gilt dies für den Patientenkreis, bei dem das Gehör durch Unfall, Krankheit oder sonstige Einflüsse vollständig ausgefallen oder bereits von Geburt an nicht funktionsfähig ist. Ist in diesen Fällen nur das Innenohr (Cochlea) und nicht die nach zentral führende neuronale Hörbahn betroffen, kann mit elektrischen Reizsignalen der verbliebene Hörnerv stimuliert und somit ein Höreindruck erzeugt werden, der bis zu einem offenen Sprachverständnis führen kann. Bei diesen sogenannten Cochlea-Implantaten (CI) wird ein Reizelektroden-Array in die Cochlea eingeführt, das von einem elektronischen System (Elektronikmodul) angesteuert wird. Dieses Elektronikmodul ist hermetisch dicht und biokompatibel eingekapselt, und es wird operativ im knöchernen Bereich hinter dem Ohr (Mastoid) eingebettet. Das elektronische System enthält jedoch im wesentlichen nur Dekodier- und Treiberschaltungen für die Reizelektroden. Die akustische Schallaufnahme, die Wandlung dieses Schallsignals in elektrische Signale und deren weitere Verarbeitung erfolgen grundsätzlich extern in einem sogenannten Sprachprozessor, der außen am Körper getragen wird. Der Sprachprozessor setzt die vorverarbeiteten Signale entsprechend kodiert auf ein hochfrequentes Trägersignal um, das über eine induktive Kopplung durch die geschlossene Haut (transkutan) zu dem Implantat übertragen wird. Das schallaufnehmende Mikrofon befindet sich ausnahmslos außerhalb des Körpers und in den meisten Anwendungen in einem an der Ohrmuschel getragenen Gehäuse eines Hinter-dem-Ohr-Hörgerätes (HdO), und es ist mit einem Kabel mit dem Sprachprozessor verbunden. Solche Cochlea-Implantat-Systeme, deren Komponenten und Prinzipien der transkutanen Signalübertragung sind beispielhaft in folgenden Patentschriften beschrieben: US-A-5 070 535, US-A-4 441 210, EP-A-0 200 321, US-A-5 626 629, US-A-5 545 219, US-A-5 578 084, US-A-5 800 475, US-A-5 957 958, US-A-6 038 484. Verfahren zur Sprachaufbereitung und -Kodierung bei Cochlea-Implantaten sind beispielsweise in folgenden Patentschriften angegeben: EP-A-0 823 188, EP-A-0 190 836, US-A-5 597 380, US-A-5 271 397, US-A-5 095 904, US-A-5 601 617, US-A-5 603 726. Neben der Rehabilitation gehörloser beziehungsweise ertaubter Patienten mit Cochlea-Implantaten existieren seit geraumer Zeit Ansätze, Patienten mit einer sensorineuralen Hörstörung, die operativ nicht behebbar ist, mit teilbeziehungsweise vollimplantierbaren Hörgeräten eine bessere Rehabilitation als mit konventionellen Hörgeräten zu bieten. Das Prinzip besteht in den überwiegenden Ausführungsformen darin, ein Ossikel des Mittelohres oder das Innenohr direkt über einen mechanischen beziehungsweise hydromechanischen Reiz zu stimulieren und nicht über das verstärkte akustische Signal eines konventionellen Hörgerätes, bei dem das verstärkte Schallsignal dem äußeren Gehörgang zugeführt wird. Der aktorische Stimulus dieser elektromechanischen Systeme wird mit verschiedenen physikalischen Wandlerprinzipien realisiert wie zum Beispiel durch elektromagnetische und piezoelektrische Systeme. Der Vorteil dieser Geräte wird hauptsächlich in der gegenüber konventionellen Hörgeräten verbesserten Klangqualität und bei vollimplantierten Systemen in der Unsichtbarkeit der Hörprothese gesehen. Solche teil- und vollimplantierbaren elektromechanischen Hörgeräte sind beispielhaft von Yanigahara et al. "lmplantable Hearing Aid", Arch Otolaryngol Head Neck Surg - Vol 113, 1987, 869-872; Suzuki et al. "Implantation of Partially Implantable Middle Ear Implant and the Indication" Advances in Audiology, Vol. 4, 160 166, Karger Basel, 1988; H.P. Zenner et al. "Erste Implantationen eines vollständig implantierbaren elektronischen Hörsystems bei Patienten mit Innenohrschwerhörigkeit", HNO 46:844-852; H. Leysieffer et al. "Ein vollständig implantierbares Hörsystem für Innenohrschwerhörige: TICA LZ 3001" HNO 46:853-863; H.P. Zenner et al. "Aktive elektronische Hörimplantate für Mittel- und Innenohrschwerhörige - eine neue Ära der Ohrchirurgie", HNO 45:749-774; H.P. Zenner et al. "Totally implantable hearing device for sensorineural hearing loss", The Lancet Vol. 352, No. 9142, Seite 1751; und in zahlreichen Patentschriften beschrieben, so unter anderem in: EP-A-0 263 254, EP-A-0 400 630, EP-A-0 499 940, US-A-3 557 775, US-A-3 712 962, US-A-3 764 748, US-A-5 411 467, US-A-4 352 960, US-A-4 988 333, US-A-5 01 5 224, US-A-5 015 225, US-A-5 360 388, US-A-5 772 575, US-A-5 814 095, US-A-5 951 601, US-A-5 977 689 und US-A-5 984 859. Dabei ist das Einbringen eines elektromechanischen Wandlers durch eine Eröffnung im Promontorium zur direkten Flüssigkeitsanregung im Innenohr in US-A-5 772 575, US-A-5 951 601, US-A-5 977 689 und US-A-5 984 859 beschrieben.

In WO 99/08480 ist ein mindestens teilweise implantierbares System zur Rehabilitation einer Hörstörung gezeigt, welches ein Mikrofon zur Aufnahme des Schallsignals und dessen Umwandlung in entsprechende elektrische Signale, eine elektronische Signalverarbeitungseinheit zur Audiosignalverarbeitung und -verstärkung, eine elektrische Energieversorgungseinheit, die einzelne Komponenten des Systems mit Strom versorgt sowie eine ausgangsseitige aktorische Stimulationsanordnung umfasst. Des weiteren offenbart die WO 99/08480 ein implantierbares System zur Rehabilitation einer Hörstörung, bei welchem ein Cochlear-Implantat zur elektrischen Stimulation des Innenohres vorgesehen ist, welches in Kombination mit einem System zur Erfassung von Vibrationen im Mittelohr vorgesehen ist.

Viele Patienten mit einem Innenohrschaden leiden zusätzlich unter zeitweise auftretenden oder permanenten Ohrgeräuschen (Tinnitus), die operativ nicht behebbar sind und gegen die bis heute keine zugelassenen medikamentösen Behandlungsformen existieren. Daher sind sogenannte Tinnitus-Maskierer bekannt (WO-A-90/07251, EP-A-0 537 385, DE-U 296 16 956). Dies sind kleine, batteriebetriebene Geräte, die ähnlich einem Hörgerät hinter oder im Ohr getragen werden und durch artifizielle Schalle, die beispielsweise über einen Hörgeräte-Lautsprecher in den Gehörgang abgestrahlt werden, den Tinnitus auf psychoakustisch wirkende Weise verdecken ("maskieren") und das störende Ohrgeräusch so möglichst unter die Wahrnehmungsschwelle absenken. Die artifiziellen Schalle sind häufig Schmalbandgeräusche (zum Beispiel Terzrauschen), die in ihrer spektralen Lage und ihrem Lautstärkepegel über ein Programmiergerät einstellbar sind, um eine möglichst optimale Anpassung an die individuelle Ohrgeräuschsituation zu ermöglichen. Darüber hinaus existiert seit kurzem die sog. "Retraining-Methode", wobei durch die Kombination eines mentalen Trainingsprogrammes und die Darbietung eines breitbandigen Schalles (Rauschen) nahe der Ruhehörschwelle die Wahrnehmbarkeit des Tinnitus ebenfalls weitgehend unterdrückt werden soll (H. Knör, "Tinnitus-Retraining-Therapie und Hörakustik" Zeitschrift "Hörakustik" 2/97, Seiten 26 und 27). Diese Geräte werden auch als "Noiser" bezeichnet.

Bei beiden oben genannten Methoden zur apparativen Therapie des Tinnitus sind hörgeräteähnliche, technische Geräte außen am Körper im Ohrbereich sichtbar mitzuführen, die den Träger stigmatisieren und daher nicht gerne getragen werden.

In US-A-5 795 287 wird ein implantierbarer Tinnitusmaskierer mit "Direktantrieb" ("direct drive") des Mittelohres zum Beispiel über einen an die Ossikelkette angekoppelten elektromechanischen Wandler beschrieben. Dieser direkt gekoppelte Wandler kann vorzugsweise ein sogenannter "Floating Mass Transducer" (FMT) sein. Dieser FMT entspricht dem Wandler für implantierbare Hörgeräte, der in US-A-5 624 376 beschrieben ist.

In DE-C-198 58 398 und EP 1 014 755 werden implantierbare Systeme zur Behandlung eines Tinnitus durch Maskierung und/oder Noiserfunktionen beschrieben, bei denen der signalverarbeitende elektronische Pfad eines teil oder vollimplantierbaren Hörsystems durch entsprechende elektronische Module so ergänzt wird, dass die zur Tinnitus-maskierung oder zur Noiserfunktion notwendigen Signale in den Signalverarbeitungsweg der Hörgerätefunktion eingespeist und die zugehörigen Signalparameter durch weitere elektronische Maßnahmen individuell an die pathologischen Bedürfnisse angepasst werden können. Diese Anpassbarkeit kann dadurch realisiert werden, dass die notwendigen Einstelldaten der Signalerzeugungs- und Einspeiselektronik in demselben physikalischen und logischen Datenspeicherbereich des Implantatsystems hard- und softwaremäßig abgelegt beziehungsweise programmiert werden und über entsprechende elektronische Stellglieder die Einspeisung des Maskierer- beziehungsweise Noisersignals in den Audiopfad des Hörimplantats steuern.

Implantierbare Rehabilitationsgeräte der vorstehend genannten Art bestehen je nach angestrebter Funktion aus mehreren Funktionseinheiten, insbesondere einem Sensor (Mikrofon), der den einfallenden Luftschall in ein elektrisches Signal umwandelt, einer elektronischen Signalverarbeitungs-, Verstärkungs- und Implantatsteuerungseinheit, einem implantierbaren elektromechanischen bzw. elektroakustischen Wandler, der die verstärkten und vorverarbeiteten Sensorsignale in mechanische bzw. akustische Schwingungen umwandelt und diese über geeignete Koppelmechanismen dem geschädigten Mittel- und/oder Innenohr zuführt, oder einer cochleären Reizelektrode bei Cochlea-Implantaten, sowie einem elektrischen Energieversorgungssystem, das die genannten Module versorgt. Weiterhin kann eine externe Einheit vorgesehen sein, die dem Implantat elektrische Nachladeenergie zur Verfügung stellt, wenn die implantatseitige Energieversorgungseinheit eine nachladbare (Sekundär-)Batterie enthält. Besonders vorteilhafte Vorrichtungen und Verfahren zum Laden von nachladbaren Implantatbatterien sind in DE-A-198 38 137 sowie in EP-B-0 499 939 beschrieben. Zweckmäßig ist auch eine Telemetrieeinheit vorzusehen, mit der patientenspezifische, audiologische Daten drahtlos bidirektional übertragen bzw. im Implantat programmiert und damit dauerhaft gespeichert werden können, wie es von Leysieffer et al. in HNO Vol. 46, 1998, S. 853-863 beschrieben wurde.

Grundsätzlich sind bei allen genannten, mindestens teilweise implantierbaren Systemen die (Audio-)Signalverarbeitung bzw. Signalerzeugung sowie die Module der Implantatsteuerung wie z.B. ein geregeltes Akkumulator-Nachladesystem oder ein Telemetriesystem zur bidirektionalen Übertragung von z.B. veränderlichen, patientenspezifischen Parametern implantatseitig durch fest vorgegebene Hardwareeinheiten realisiert. Dies gilt auch dann, wenn zur Signalverarbeitung bzw. -erzeugung oder zum Implantatmanagement digitale Signalprozessoren oder Microcontroller bzw. Mikroprozessoren eingesetzt werden, unabhängig davon, ob diese als eine sogenannte "hardwired logic", d.h. in "festverdrahteter" Logikarchitektur, realisiert sind oder ob deren Betriebsprogramme in Festwertspeicherbereichen (z.B. ROM) der entsprechenden Prozessoren abgelegt sind. Diese Programme, die zum grundlegenden Betrieb des Implantats sowie zur bestimmungsgemäßen Funktion notwendig und vorgesehen sind, werden im folgenden als Betriebsprogramm bzw. als Betriebssoftware bezeichnet. Diese Betriebssoftware wird bei den bekannten Implantatsystemen während der Produktion zum Beispiel durch Maskenprogrammierung der Prozessor-Speicherbereiche in das System eingebracht, und sie ist nach der Implantation nicht mehr veränderbar.

Im Gegensatz dazu werden patientenspezifische Daten wie z.B. audiologische Anpassdaten oder auch veränderbare Implantatsystemparameter (z.B. als Variable in einem der oben genannten Softwareprogramme zur Regelung einer Akkumulatornachladung) vorliegend als Betriebsparameter bezeichnet. Diese Betriebsparameter können bei bekannten vollimplantierbaren Implantatsystemen nach der Implantation transkutan, d.h., drahtlos durch die geschlossene Haut, in das Implantat übertragen und damit verändert werden.

Die oben beschriebenen mindestens teilweise implantierbaren Hörsysteme zur Rehabilitation einer Innenohrschädigung, die auf einem ausgangsseitigen elektromechanischen Wandler basieren, unterscheiden sich von herkömmlichen Hörgeräten im wesentlichen nur dadurch, dass der ausgangsseitige akustische Stimulus (das heißt ein verstärktes Schallsignal vor dem Trommelfell) durch einen verstärkten mechanischen Stimulus des Mittel- beziehungsweise Innenohres ersetzt wird. Der akustische Stimulus eines konventionellen Hörgerätes führt schließlich über die mechanische Anregung des Trommelfells und des sich anschließenden Mittelohres auch zu einem vibratorischen, d.h. mechanischen Reiz des Innenohres. Bezüglich der sinnvollen Audiosignalvorverarbeitung bestehen grundlegend ähnliche beziehungsweise gleiche Anforderungen. Weiterhin wird in beiden Ausführungsformen letztendlich ausgangsseitig ein örtlich lokalisierter vibratorischer Stimulus an das geschädigte Innenohr geleitet (zum Beispiel verstärkte mechanische Schwingung des Steigbügels im ovalen Fenster des Innenohres).

Bei den Cochlea Implantaten werden ausschließlich elektrische Reizsignale verwendet. Nach der Implantation eines CI bei völlig ertaubten bzw. gehörlosen Patienten ist im Regelfall ein Training zur Hörrehabilitation notwendig, da die artifiziellen Reize gelernt werden müssen, weil sie prinzipiell nicht der biologisch adäquaten Reizform des Innenohres entsprechen. Dem gegenüber entfällt diese Rehabilitationsphase nach Implantation eines elektromechanischen Hörsystems bei Schwerhörigen, da die mechanische Reizform, wie oben beschrieben, biologisch adäquat ist und letztendlich einer Versorgung mit einem Hörgerät zumindest bezüglich der grundsätzlichen Funktion weitgehend entspricht, das heißt, der stimulierende Reiz am Eingang des Innenohres ist vibratorischer Natur.

Seit kurzem sind teil- und vollimplantierbare Hörsysteme zur Rehabilitation eines Innenohrschadens in der klinischen Anwendung. Dabei zeigt sich je nach verwendetem physikalischem Prinzip des ausgangsseitigen elektromechanischen Wandlers und insbesondere dessen Ankopplungsart an die Ossikel des Mittelohres, dass die erreichten Ergebnisse der Verbesserung des Sprachverständnisses sehr unterschiedlich sein können. Dazu kommt, dass bei manchen Patienten kein ausreichender Lautstärkepegel erreicht werden kann. Dieser Aspekt ist spektral sehr unterschiedlich, was bedeuten kann, dass bei zum Beispiel mittleren und hohen Frequenzen die erzeugte Lautheit zwar ausreichend ist, jedoch nicht bei tiefen Frequenzen beziehungsweise umgekehrt. Weiterhin kann die übertragbare spektrale Bandbreite begrenzt sein, so zum Beispiel bei elektromagnetischen Wandlern auf tiefe und mittlere Frequenzen oder bei piezoelektrischen Wandlern auf mittlere und hohe Frequenzen. Darüber hinaus können sich nichtlineare Verzerrungen, die insbesondere bei elektromagnetischen Wandlern ausgeprägt sind, negativ auf die resultierende Klangqualität auswirken. Die mangelnde Lautheit führt insbesondere dazu, dass der audiologische Indikationsbereich für die Implantation eines elektromechanischen Hörsystems sehr eingeschränkt sein kann. Das bedeutet, dass Patienten beispielsweise mit einem sensorineuralen Hörverlust von größer 50 dB HL (= hearing loss, Hörverlust) im Tieftonbereich mit einem piezoelektrischen System nur unzureichend versorgt werden können. Demgegenüber sind ausgeprägte Hochtonverluste mit elektromagnetischen Wandlern nur schwer versorgbar. Bei an Taubheit grenzender Schwerhörigkeit sind aus den genannten Gründen implantierbare, elektromechanische Systeme bislang nicht einsetzbar. Hier kommen Cochlea-Implantate mit rein elektrischer Reizung des Innenohres in Frage, die naturgemäß keine Klangqualität erwarten lassen, die zum Beispiel eine akzeptable Musikübertragung ermöglicht, sondern vorrangig zur Erlangung beziehungsweise Wiederherstellung eines ausreichenden Sprachverständnisses möglichst ohne Lippenablesen konzipiert sind. Aufgrund der elektrischen Reizung sind, wie beschrieben, Hörverluste in einem spektral breiten, audiologischen Bereich möglich, die bis zur vollständigen Ertaubung reichen.

Seit kurzer Zeit ist wissenschaftlich aus CI-Implantationen bekannt, dass auch bei nicht vollständiger Taubheit CIs erfolgreich angewendet werden können, wenn mit einem konventionellen Hörgerät keine ausreichende Sprachdiskrimination mehr erreicht werden kann. Interessanterweise konnte nachgewiesen werden, dass die wesentlichen Innenohrstrukturen, die die akustische Resthörigkeit ermöglichen, zum Teil oder weitgehend langzeitstabil erhalten werden können, wenn eine CI-Elektrode in die Cochlea eingeführt wird (Ruh, S. et al.: "Cochlear Implant bei resthörigen Patienten", Laryngo-Rhino-Otol. 76 (1997), 347-350; Müller-Deile, J. et al.: "Cochlea-Implant-Versorgung bei nicht tauben Patienten?", Laryngo-Rhino-Otol. 77 (1998), 136-143; Lehnhardt, E.:"Intracochlear placement of cochlea implant electrodes in soft surgery technique", HNO 41 (1993), 356-359). In absehbarer Zeit werden CI-Elektroden bei Resthörigkeit klinisch sicher so intracochleär plaziert werden können, dass die verbleibenden Innenohrstrukturen langzeitstabil erhaltbar sind und somit auch auf biologisch adäquatem Weg, das heißt vibratorisch, weiterhin stimulierbar sind und zu einem verwertbaren Höreindruck führen.

Der Erfindung liegt die Aufgabe zugrunde, ein gegenüber den erläuterten bekannten Geräten verbessertes, mindestens teilweise implantierbares System zur Rehabilitation einer Hörstörung zu schaffen, das besonders gut und flexibel an die individuelle pathologische und audiologische Situation des jeweiligen Patienten angepasst werden kann.

Diese Aufgabe wird dadurch gelöst, dass bei einem mindestens teilweise implantierbaren System zur Rehabilitation einer Hörstörung, das mindestens einen Sensor (Mikrofon) zur Aufnahme des Schallsignals und dessen Umwandlung in entsprechende elektrische Signale, eine elektronische Signalverarbeitungseinheit zur Audiosignalverarbeitung und -verstärkung, eine elektrische Energieversorgungseinheit, die einzelne Komponenten des Systems mit Strom versorgt, sowie eine ausgangsseitige aktorische Stimulationsanordnung umfasst, erfindungsgemäß die ausgangsseitige Stimulationsanordnung in Kombination einen elektromechanischen Wandler zur mechanischen Stimulation des Mittel- oder Innenohres und ein intracochleäres, elektrisch wirkendes Reizelektrodenarray mit mindestens einer Reizelektrode zur elektrischen Stimulation des Innenohres aufweist.

Die oben genannten Nachteile der heute verfügbaren implantierbaren, elektromechanischen Hörsysteme einerseits und die Nachteile der Cochlea-Implantate andererseits werden durch vorliegende Erfindung zumindest teilweise umgangen, weil beide Stimulationsarten, d.h. mechanische und elektrische Reize, in einem einzigen Implanitat-System zur Anwendung kommen und je nach individueller pathologischer und audiologischer Situation patientenspezifisch appliziert werden können. Ein solches System kann als "duales" Hörimplantat bezeichnet werden. Weiterhin wird mit dem vorliegenden Hörimplantatsystem auch ein zumindest peripher zu lokalisierender Tinnitus effektiver maskierbar sein als mit bekannten, konventionellen Tinnitus-Maskern.

Vorteilhafte weitere Ausgestaltungen der Erfindung ergeben sich aus den Unteransprüchen.

Der ausgangsseitige elektromechanische Wandler ist bevorzugt hermetisch dicht aufgebaut, und er kann grundsätzlich nach jedem bekannten elektromechanischen Wandlerprinzip arbeiten und insbesondere als elektromagnetischer, elektrodynamischer, piezoelektrischer, magnetostriktiver oder dielektrischer (kapazitiver) Wandler ausgebildet sein. Das piezoelektrische Prinzip und das dielektrische beziehungsweise kapazitive Prinzip sind besonders bevorzugt. Bei Anwendung des piezoelektrischen Wandlerprinzips ist der Wandler vorteilhaft unter Verwendung von PZT-Keramik (Blei-Zirkonat-Titanat) oder von PVDF (Polyvinylidenfluorid) aufgebaut.

Der ausgangsseitige elektromechanische Wandler kann für eine direkte mechanische Kopplung mit dem Mittelohr oder für eine berührungslose, luftspaltgekoppelte Ankopplung an das Mittelohr nach dem elektromagnetischen Wandlerprinzip (Permanentmagnet an Ossikel angebracht) ausgelegt sein, um seinen vibratorischen Ausgangsstimulus an ein Ossikel des Mittelohres zu übertragen.

Der elektromechanische Wandler kann aber auch für eine direkte hydromechanische Kopplung mit dem Innenohr ausgelegt sein, wobei die Übertragung des vibratorischen Ausgangsstimulus an das Innenohr durch direkte mechanische Stimulation der lymphatischen Innenohrräume über einen Zugang durch das ovale, das runde oder ein artifizielles cochleäres Fenster erfolgen kann. Eine solche direkte Stimulation der Cochlea hat den Vorteil, dass das Auftreten von Rückkopplungen, das heißt die Einkopplung des Ausgangssignals in den Sensor (Mikrofon), vermieden oder weitgehend reduziert wird, weil die Ossikelkette und damit das Trommelfell nicht beziehungsweise deutlich reduziert zu Schwingungen angeregt wird. Dies ist insbesondere dann von Vorteil, wenn ein Schallsensor (Mikrofonfunktion) in unmittelbarer Nähe zum Trommelfell appliziert wird, wie dies aus US-A-5 814 095 und US-A-5 999 632 bekannt ist.

Der ausgangsseitige elektromechanische Wandler hat zweckmäßig einen Übertragungsbereich von etwa 100 Hz bis etwa 10 kHz, und er ist vorzugsweise hoch abgestimmt, das heißt, seine erste mechanische Resonsanzfrequenz liegt am oberen Ende des angestrebten Übertragungsfrequenzbereiches, insbesondere bei etwa 8 kHz bis etwa 10 kHz. Dadurch wird erreicht, dass der Auslenkungsfrequenzgang des Wandlers im Übertragungsbereich weitgehend frei ist von Resonanzen und bei Spannungseinprägung und Verwendung piezoelektrischer Wandler frequenzunabhängig eben. Im Übertragungsbereich tritt somit keine Welligkeit auf.

Die Signalverarbeitungseinheit weist zweckmäßig eine Vorverarbeitungsanordnung zum Vorverstärken und/oder Filtern sowie zum Analog-Digital- (A/D-) Wandeln der Schallsensorsignale auf. Sie kann insbesondere einen Antialiasing-Filter umfassen. Bei Vorhandensein mehrerer Schallsensoren ist vorzugsweise jedem der Schallsensoren ein eigener Analog-Digital-Wandler nachgeschaltet.

In weiterer Ausgestaltung der Erfindung kann die Signalverarbeitungseinheit Softwaremodule enthalten, die parallel zum Hörgerätebetrieb die Maskierung eines Tinnitus ermöglichen. Mit dem vorliegenden dualen Hörimplantatsystem kann ein zumindest peripher zu lokalisierender Tinnitus effektiver maskiert werden als mit bekannten, konventionellen Tinnitus-Maskern.

Die Signalverarbeitungseinheit weist vorteilhaft einen digitalen Signalprozessor zum Verarbeiten der A/D-gewandelten und gegebenenfalls mittels der Vorverarbeitungsanordnung vorverarbeiteten Schallsensorsignale und/oder zum Generieren von digitalen Signalen für eine Tinnitusmaskierung auf, wobei der ausgangsseitigen Stimulationsanordnung mindestens ein Digital/Analog-Wandlerzugeordnet ist und vorzugsweise dem ausgangsseitigen elektromechanischen Wandler und der oder den Elektrode(n) des Reizelektrodenarrays jeweils ein eigener Digital Analog-Wandler vorgeschaltet ist.

In weiterer Ausgestaltung der Erfindung enthält der digitale Signalprozessor Softwaremodule, welche die Ansteuerung des ausgangsseitigen elektromechanischen Wandlers und des Reizelektrodenarrays so vornehmen, dass die spektralen, zeitlichen, amplituden- und phasenbezogenen Wandler- beziehungsweise Reizelektroden-Signaleigenschaften derart konfiguriert sind, dass patientenspezifisch ein optimaler Hörerfolg erzielt wird.

Die Softwaremodule können dabei statisch in der Weise ausgelegt sein, dass sie aufgrund wissenschaftlicher Erkenntnisse einmalig in einem Programmspeicher des digitalen Signalprozessors abgelegt werden und unverändert bleiben. Liegen dann aber später zum Beispiel aufgrund neuerer wissenschaftlicher Erkenntnisse verbesserte Algorithmen zur Sprachsignalaufbereitung und -verarbeitung vor und sollen diese genutzt werden, muss durch einen invasiven, operativen Patienteneingriff das gesamte Implantat oder das Implantatmodul, das die entsprechende Signalverarbeitungseinheit enthält, gegen ein neues mit der veränderten Betriebssoftware ausgetauscht werden. Dieser Eingriff birgt erneute medizinische Risiken für den Patienten und ist mit hohem Aufwand verbunden.

Diesem Problem kann dadurch begegnet werden, dass in weiterer Ausgestaltung der Erfindung eine drahtlose, bevorzugt PC-basierte, Telemetrieeinrichtung zur Übertragung von Daten zwischen dem implantierten Teil des Systems und einer externen Einheit, insbesondere einem externen Programmiersystem vorgesehen ist, wobei vorzugsweise dem Signalprozessor zur Aufnahme und Wiedergabe eines Betriebsprogramms eine wiederholt beschreibbare, implantierbare Speicheranordnung zugeordnet ist und mindestens Teile des Betriebsprogramms durch von der externen Einheit über die Telemetrieeinrichtung übermittelte Daten geändert oder ausgetauscht werden können. Auf diese Weise lässt sich nach Implantation des implantierbaren Systems die Betriebssoftware als solche verändern oder auch vollständig austauschen, wie dies für im übrigen bekannte Systeme zur Rehabilitation von Hörstörungen in DE 199 15 846.0 erläutert ist.

Bevorzugt ist die Auslegung so beschaffen, dass darüber hinaus bei vollimplantierbaren Systemen auch in an sich bekannter Weise Betriebsparameter, das heißt patientenspezifische Daten, wie beispielsweise audiologische Anpassdaten, oder veränderbare Implantatsystemparameter (zum Beispiel als Variable in einem Softwareprogramm zur Regelung einer Batterienachladung) nach der Implantation transkutan, das heißt drahtlos durch die geschlossene Haut, in das Implantat übertragen und damit verändert werden können. Dabei sind die Softwaremodule bevorzugt dynamisch, oder mit anderen Worten lernfähig, ausgelegt, um zu einer möglichst optimalen Rehabilitation der jeweiligen Hörstörung zu kommen. Insbesondere können die Softwaremodule adaptiv ausgelegt sein, und eine Parameteranpassung kann durch "Training" durch den Implantatträger und weitere Hilfsmittel vorgenommen werden.

Weiterhin kann die Signalverarbeitungselektronik ein Softwaremodul enthalten, das eine möglichst optimale Stimulation auf der Basis eines lernfähigen neuronalen Netzwerkes erreicht. Das Training dieses neuronalen Netzwerks kann wieder durch den Implantatträger erfolgen und/oder unter Zuhilfenahme weiterer externer Hilfsmittel.

Die Speicheranordnung zum Speichern von Betriebsparametern und die Speicheranordnung zur Aufnahme und Wiedergabe des Betriebsprogramms können als voneinander unabhängige Speicher implementiert sein; es kann sich jedoch auch um einen einzigen Speicher handeln, in dem sowohl Betriebsparameter als auch Betriebsprogramme abgelegt werden können.

Die vorliegende Lösung erlaubt eine Anpassung des Systems an Gegebenheiten, die erst nach Implantation des implantierbaren Systems erfassbar sind. So sind beispielsweise bei einem mindestens teilweise implantierbaren Hörsystem zur Rehabilitation einer monauralen oder binauralen Innenohrstörung sowie eines Tinnitus mit mechanischer Stimulation des Innenohres die sensorischen (Schallsensor bzw. Mikrofon) und aktorischen (Ausgangsstimulator) biologischen Schnittstellen immer abhängig von den anatomischen, biologischen und neurophysiologischen Gegebenheiten, z.B. von dem interindividuellen Einheilprozess. Diese Schnittstellenparameter können individuell insbesondere auch zeitvariant sein. So können beispielsweise das Übertragungsverhalten eines implantierten Mikrofons aufgrund von Gewebebelagen und das Übertragungsverhalten eines an das Innenohr angekoppelten elektromechanischen Wandlers aufgrund unterschiedlicher Ankopplungsqualität interindividuell und individuell variieren. Solche Unterschiede der Schnittstellenparameter, die sich bei den aus dem Stand der Technik bekannten Vorrichtungen nicht einmal durch den Austausch des Implantats mindern beziehungsweise eliminieren ließen, können vorliegend durch Veränderung bzw. Verbesserung der Signalverarbeitung des Implantats optimiert werden.

Bei einem mindestens teilweise implantierbaren Hörsystem kann es sinnvoll oder notwendig werden, nach Implantation verbesserte Signalverarbeitungsalgorithmen zu implementieren. Dabei sind insbesondere zu nennen:
- Sprachanalyseverfahren (z.B. Optimierung einer Fast-Fourier-Transformation (FFT)),
- statische oder adaptive Störschallerkennungsverfahren,
- statische oder adaptive Störschallunterdrückungsverfahren,
- Verfahren zur Optimierung des systeminternen Signal-Rauschabstandes,
- optimierte Signalverarbeitungsstrategien bei progredienter Hörstörung,
- ausgangspegelbegrenzende Verfahren zum Schutz des Patienten bei Implantatfehlfunktionen bzw. externen Fehlprogrammierungen,
- Verfahren zur Vorverarbeitung mehrerer Sensor(Mikrofon)signale, insbesondere bei binauraler Positionierung der Sensoren,
- Verfahren zur binauralen Verarbeitung zweier oder mehrerer Sensorsignale bei binauraler Sensorpositionierung, z.B. Optimierung des räumlichen Hörens bzw. Raumorientierung,
- Phasen- bzw. Gruppenlaufzeit-Optimierung bei binauraler Signalverarbeitung,
- Verfahren zur optimierten Ansteuerung der Ausgangsstimulatoren, insbesondere bei binauraler Positionierung der Stimulatoren.

Mit dem vorliegenden System lassen sich auch nach der Implantation unter anderem die folgenden Signalverarbeitungsalgorithmen implementieren:
- Verfahren zur Rückkopplungsunterdrückung bzw. -minderung,
- Verfahren zur Optimierung des Betriebsverhaltens des bzw. der Ausgangswandler (z.B. Frequenz- und Phasengangoptimierung, Verbesserung des Impulsübertragungsverhaltens),
- Sprachsignal-Kompressionsverfahren bei Innenohrschwerhörigkeiten,
- Signalverarbeitungsmethoden zur Recruitment-Kompensation bei Innenohrschwerhörigkeiten.

Des weiteren ist bei Implantatsystemen mit einer sekundären Energieversorgungseinheit, d.h. einem nachladbaren Akkumulatorsystem, aber auch bei Systemen mit primärer Batterieversorgung davon auszugehen, dass diese elektrischen Energiespeicher mit voranschreitender Technologie immer größere Lebensdauern und damit steigende Verweilzeiten im Patienten ermöglichen. Es ist davon auszugehen, dass die Grundlagen- und Applikationsforschung für Signalverarbeitungsalgorithmen schnelle Fortschritte macht. Die Notwendigkeit oder der Patientenwunsch einer Betriebssoftwareanpassung bzw. -veränderung wird daher voraussichtlich vor Ablauf der Lebensdauer der implantatintemen Energiequelle eintreten. Das vorliegend beschriebene System erlaubt eine derartige Anpassung der Betriebsprogramme des Implantats auch im bereits implantierten Zustand.

Vorzugsweise ist ferner eine Zwischenspeicheranordnung vorgesehen, in welcher von der externen Einheit über die Telemetrieeinrichtung übermittelte Daten vor dem Weiterleiten an den Signalprozessor zwischengespeichert werden können. Auf diese Weise lässt sich der Übertragungsvorgang von der externen Einheit zu dem implantierten System abschließen, bevor die über die Telemetrieeinrichtung übermittelten Daten an den Signalprozessor weitergeleitet werden.

Des weiteren kann eine Überprüfungslogik vorgesehen sein, die in der Zwischenspeicheranordnung gespeicherte Daten vor dem Weiterleiten an den Signalprozessor einer Überprüfung unterzieht. Es kann ein Mikroprozessorbaustein, insbesondere ein Microcontroller, zum implantatinternen Steuern der A/D-Wandler und/oder der D/A-Wandler und/oder des Signalprozessors über einen Datenbus vorgesehen sein, wobei zweckmäßig die Überprüfungslogik und die Zwischenspeicheranordnung in dem Mikroprozessorbaustein implementiert sind und wobei über den Datenbus und die Telemetrieeinrichtung auch Programmteile oder ganze Softwaremodule zwischen der Außenwelt, dem Mikroprozessorbaustein und dem Signalprozessor übermittelt werden können.

Dem Mikroprozessorbaustein ist vorzugsweise eine implantierbare Speicheranordnung zum Speichern eines Arbeitsprogramms für den Mikroprozessorbaustein zugeordnet, und mindestens Teile des Arbeitsprogramms für den Mikroprozessorbaustein können durch von der externen Einheit über die Telemetrieeinrichtung übermittelte Daten geändert oder ausgetauscht werden.

In weiterer Ausgestaltung der Erfindung können mindestens zwei Speicherbereiche zur Aufnahme und Wiedergabe mindestens des Betriebsprogramms des Signalprozessors vorgesehen sein. Dies trägt zur Fehlersicherheit des Systems bei, indem durch das mehrfache Vorhandensein des Speicherbereichs, welcher das beziehungsweise die Betriebsprogramme enthält, beispielsweise nach einer Übertragung von extern oder aber beim Einschalten des Implantats eine Überprüfung der Fehlerfreiheit der Software durchgeführt werden kann.

Analog hierzu kann auch die Zwischenspeicheranordnung mindestens zwei Speicherbereiche zur Aufnahme und Wiedergabe von von der externen Einheit über die Telemetrieeinrichtung übermittelten Daten aufweisen, so dass nach einer Datenübertragung von der externen Einheit noch im Bereich des Zwischenspeichers eine Überprüfung der Fehlerfreiheit der übermittelten Daten vorgenommen werden kann. Die Speicherbereiche können zur beispielsweise komplementären Ablage der von der externen Einheit übermittelten Daten ausgelegt sein. Mindestens einer der Speicherbereiche der Zwischenspeicheranordnung kann aber auch zur Aufnahme nur eines Teils der von der externen Einheit übermittelten Daten ausgelegt sein, wobei in diesem Fall die Überprüfung der Fehlerfreiheit der übermittelten Daten abschnittsweise erfolgt.

Um zu gewährleisten, dass bei Übertragungsfehlern ein erneuter Übertragungsvorgang gestartet werden kann, kann dem Signalprozessor ferner ein vorprogrammierter, nicht überschreibbarer Festspeicherbereich zugeordnet sein, in welchem die für einen "Minimalbetrieb" des Systems erforderlichen Anweisungen und Parameter gespeichert sind, beispielsweise Anweisungen, die nach einem "Systemabsturz" zumindest einen fehlerfreien Betrieb der Telemetrieeinrichtung zum Empfang eines Betriebsprogramms sowie Anweisungen zum Einspeichern desselben in die Steuerlogik gewährleisten.

Wie bereits erwähnt, ist die Telemetrieeinrichtung in vorteilhafter Weise außer zum Empfang von Betriebsprogrammen von der externen Einheit auch zur Übermittlung von Betriebsparametern zwischen dem implantierbaren Teil des Systems und der externen Einheit ausgelegt, so dass einerseits solche Parameter von einem Arzt, einem Hörgeräteakustiker oder dem Träger des Systems selbst eingestellt werden können (z.B. Lautstärke), andererseits das System aber auch Parameter an die externe Einheit übermitteln kann, beispielsweise um den Status des Systems zu überprüfen.

Ein vollständig implantierbares Hörsystem der vorliegend erläuterten Art kann implantatseitig neben der aktorischen Stimulationsanordnung und der Signalverarbeitungseinheit mindestens einen implantierbaren Schallsensor und ein nachladbares elektrisches Speicherelement aufweisen, wobei in einem solchen Fall eine drahtlose, transkutane Ladevorrichtung zum Laden des Speicherelements vorgesehen sein kann. Es versteht sich jedoch, dass zur Energieversorgung auch eine Primärzelle oder eine andere Energieversorgungseinheit vorhanden sein kann, die keine transkutane Nachladung benötigt. Dies gilt insbesondere, wenn man berücksichtigt, dass in naher Zukunft vor allem durch Weiterentwicklung der Prozessortechnologie mit wesentlicher Verminderung des Energiebedarfs für elektronische Signalverarbeitung zu rechnen ist, so dass für implantierbare Hörsysteme neue Energieversorgungsformen praktisch anwendbar werden, zum Beispiel eine den Seebeck-Effekt nutzende Energieversorgung, wie sie in DE-C 198 27 898 beschrieben ist. Vorzugsweise ist auch eine drahtlose Fernbedienung zur Steuerung der Implantatfunktionen durch den Implantatträger vorhanden.

Bei teilimplantierbarer Ausbildung des Hörsystems sind mindestens ein Schallsensor, die elektronische Signalverarbeitungseinheit, die Energieversorgungseinheit sowie eine Modulator/Sender-Einheit in einem extern am Körper, vorzugsweise am Kopfüber dem Implantat, zu tragenden externen Modul enthalten. Das Implantat weist den ausgangsseitigen elektromechanischen Wandler und das intracochleäre Reizelektrodenarray auf, ist aber energetisch passiv und empfängt seine Betriebsenergie und Wandlersteuerdaten über die Modulator/Sender-Einheit im externen Modul.

Das beschriebene System kann bei vollimplantierbarer Auslegung ebenso wie bei teilimplantierbarem Aufbau monaural oder binaural ausgelegt sein. Ein binaurales System zur Rehabilitation einer Hörstörung beider Ohren weist zwei Systemeinheiten auf, die jeweils einem der beiden Ohren zugeordnet sind. Dabei können die beiden Systemeinheiten einander im wesentlichen gleich sein. Es kann aber auch die eine Systemeinheit als Master-Einheit und die andere Systemeinheit als von der Master-Einheit gesteuerte Slave-Einheit ausgelegt sein. Die Signalverarbeitungsmodule der beiden Systemeinheiten können auf beliebige Weise, insbesondere über eine drahtgebundene implantierbare Leitungsverbindung oder über eine drahtlose Verbindung, vorzugsweise eine bidirektionale Hochfrequenzstrecke, eine körperschallgekoppelte Ultraschallstrecke oder eine die elektrische Leitfähigkeit des Gewebes des Implantatträgers ausnutzende Datenübertragungsstrecke, so miteinander kommunizieren, dass in beiden Systemeinheiten eine optimierte binaurale Signalverarbeitung und Wandler-Array-Ansteuerung erreicht wird.

Bevorzugte Ausführungsformen des erfindungsgemäßen Systems sind nachstehend unter Bezugnahme auf die beiliegenden Zeichnungen näher beschrieben. Es zeigen:
- Fig. 1: schematisch ein Ausführungsbeispiel für den Aufbau eines vollständig implantierbaren dualen Hörsystems nach der Erfindung,
- Fig. 2: schematisch ein Ausführungsbeispiel für den Aufbau eines signalverarbeitenden Elektronikmoduls eines mindestens teilweise implantierbaren Hörsystems,
- Fig. 3: eine binaurale Anwendung eines Hörimplantats gemäß Fig. 1, bei dem die Signalverarbeitungsmodule über eine drahtgebundene implantierbare Leitungsverbindung miteinander kommunizieren,
- Fig. 4: eine binaurale Anwendung eines Hörimplantats gemäß Fig. 1, bei dem die Signalverarbeitungsmodule über eine drahtlose Verbindung miteinander kommunizieren,
- Fig. 5: eine binaurale Anwendung eines Hörimplantats gemäß Fig. 1, bei dem die Signalverarbeitungsmodule über eine körperschallgekoppelte Ultraschallstrecke miteinander kommunizieren, sowie
- Fig. 6: eine binaurale Anwendung eines Hörimplantats gemäß Fig. 1, bei dem die Signalverarbeitungsmodule über eine das Gewebe des Implantatträgers einschließende Übertragungsstrecke miteinander kommunizieren, und
- Fig. 7: schematisch ein Ausführungsbeispiel für den Aufbau eines teilimplantierbaren Hörsystems mit einer Stimulationsanordnung gemäß Fig. 1.

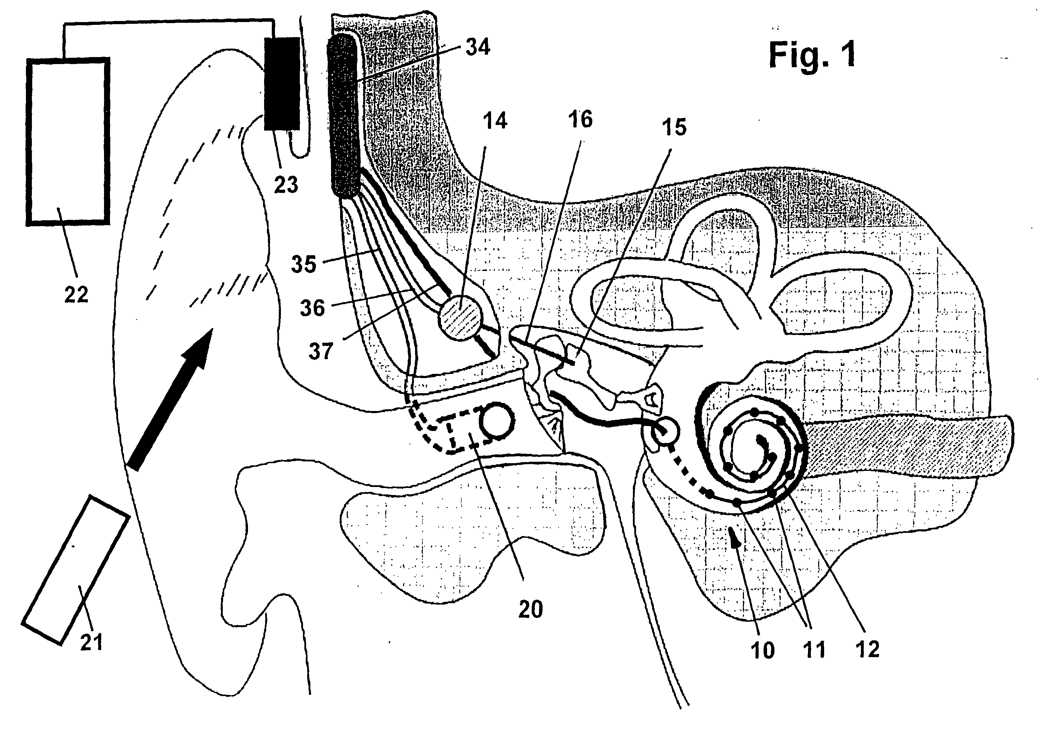

Fig. 1 zeigt schematisch den Aufbau eines vollständig implantierbaren Hörsystems, dessen ausgangsseitige aktorische Stimulationsanordnung ein elektrisches, intracochleäres Array 10 mit mehreren Reizelektroden 11 und einen elektromechanischen Wandler 14 aufweist, der hier beispielhaft an den Amboss 15 angekoppelt ist.

Das intracochleäre Reizelektrodenarray 10 kann in beliebiger für Cochlea-Implantate bekannter Weise zum Beispiel als unipolare oder bipolare Anordnung aufgebaut sein. Es weist einen Elektrodenträger 12 aus elektrisch isolierendem, flexiblem Werkstoff auf, entlang dem die an Zuleitungen 13 angeschlossenen Reizelektroden 11 in gegenseitigem Abstand verteilt angeordnet sind. Die Reizelektroden 11 sind so in den Träger 12 eingebettet oder an dem Träger 12 fixiert, dass ein Oberflächenanteil je Reizelektrode in direktem galvanischem Kontakt zu der lymphatischen Flüssigkeit des Innenohres oder direkt zu einer der zu reizenden neuronalen Strukturen steht.

Der Wandler 14 kann insbesondere in der aus EP-A-0 499 940 bekannten Weise so aufgebaut sein, dass eine Gehäusewand eines hermetisch dichten Wandlergehäuses als schwingfähige Membran ausgeführt ist, die zusammen mit einer auf ihrer Innenseite aufgebrachten piezoelektrischen Keramikscheibe ein elektromechanisch aktives Heteromorph-Verbundelement darstellt und deren mechanische Schwingungen über eine auf der Außenseite der Membran fest angebrachte Koppelstange 16 und gegebenenfalls ein mit der Koppelstange verbundenes Koppelelement auf die Ossikelkette übertragen werden. Dieser Wandler kann in der in DE-C-198 40 211 erläuterten Weise dahingehend modifiziert sein, dass an der Innenseite der piezoelektrischen Keramikscheibe ein Permanentmagnet angebracht ist, der nach Art eines elektromagnetischen Wandlers mit einer Elektromagnetspule zusammenwirkt. Ein solcher kombinierter piezoelektrischer elektromagnetischer Wandler ist besonders im Hinblick auf ein breites Frequenzband und die Erzielung relativ großer Schwingungsamplituden mit verhältnismäßig kleiner zugeführter Energie von Vorteil.

Bei dem Wandler 14 kann es sich auch um eine elektromagnetische Wandleranordnung handeln, wie sie in EP-A-0 984 663 beschrieben ist, das heißt eine mit einem am Implantationsort mit Bezug auf den Schädel fixierbaren Gehäuse und einem mit Bezug auf das Gehäuse beweglichen, mechanisch steifen Koppelelement versehene Wandleranordnung, bei der in dem Gehäuse ein elektromechanischer Wandler untergebracht ist, mit dem sich das Koppelelement in Schwingungen versetzen lässt, und der als Elektromagnetanordnung ausgebildet ist, die ein mit Bezug auf das Gehäuse fixiertes Bauteil sowie ein schwingfähiges Bauteil aufweist, welches mit dem Koppelelement derart in Verbindung steht, dass Schwingungen des schwingfähigen Bauteils auf das Koppelelement übertragen werden.

Zum Ankoppeln des elektromechanischen Wandlers an das Mittel- oder Innenohr eignen sich besonders Koppelanordnungen gemäß US-A-5 941 814, bei denen ein Koppelelement außer einem Ankoppelteil für den betreffenden Ankoppelort eine Crimphülse aufweist, die zunächst lose auf einen mit rauher Oberfläche versehenen stabförmigen Teil einer Koppelstange aufgeschoben ist, die in der zuvor erläuterten Weise mit dem Wandler verbunden ist. Beim Implantieren kann die Crimphülse gegenüber der Koppelstange einfach verschoben und gedreht werden, um das Ankoppelteil des Koppelelementes mit dem beabsichtigten Ankoppelort exakt auszurichten. Dann wird die Crimphülse fixiert, indem sie mittels eines Crimpwerkzeuges plastisch kaltverformt wird. Alternativ kann das Koppelelement mit Bezug auf die Koppelstange auch mittels einer zuziehbaren Bandschlaufe festgelegt werden.

Weitere vorliegend bevorzugt verwendbare Koppelanordnungen sind im einzelnen in DE 199 23 403, DE 199 35 029, DE 199 31 788 und den älteren europäischen Patentanmeldungen 00 119 019.8 und 00 118 996.8 beschrieben. So kann gemäß DE 199 23 403 das Koppelelement an seinem Ankoppelende eine Kontaktfläche aufweisen, die eine an die Oberflächenform der Ankoppelstelle anpassbare oder angepasste Oberflächenform sowie eine solche Oberflächenbeschaffenheit und Oberflächengröße aufweist, dass es durch Anlegen des Ankoppelendes an die Ankoppelstelle zu einer dynamischen Zug-Druck-Kraftkopplung von Koppelelement und Ossikelkette durch Oberflächenadhäsion kommt, die für eine sichere gegenseitige Verbindung von Koppelelement und Ossikelkette ausreicht. Das Koppelelement kann mit einem im implantierten Zustand an der Ankoppelstelle anliegenden Dämpfungsglied mit entropieelastischen Eigenschaften versehen sein, um eine optimale Schwingungsform der Steigbügelfußplatte oder einer das runde Fenster oder ein artifizielles Fenster in der Cochlea, im Vestibulum oder im Labyrinth abschließenden Membran zu erreichen und das Risiko einer Beschädigung der natürlichen Strukturen im Bereich der Ankoppelstelle während und nach der Implantation besonders gering zu halten (DE 199 35 029).

Das Koppelelement kann entsprechend DE 199 31 788 mit einer Stellvorrichtung zum wahlweisen Verstellen des Koppelelements zwischen einer Offenstellung, in welcher das Koppelelement in und außer Eingriff mit der Ankoppelstelle bringbar ist, und einer Schließstellung versehen sein, in welcher das Koppelelement im implantierten Zustand mit der Ankoppelstelle in Kraft- und/oder Formschlussverbindung steht.

Zum mechanischen Ankoppeln des elektromechanischen Wandlers an eine vorgewählte Ankoppelstelle an der Ossikelkette, der Steigbügelfußplatte oder einer das runde Fenster oder ein artifizielles Fenster in der Cochlea, im Vestibulum oder im Labyrinth (Gleichgewichtsorgan) abschließenden Membran eignet sich ferner eine Koppelanordnung (EP-Patentanmeldung 00 119 019.8), die eine von dem Wandler in mechanische Schwingungen versetzbare Koppelstange sowie ein mit der vorgewählten Ankoppelstelle in Verbindung bringbares Koppelelement aufweist, wobei die Koppelstange und das Koppelelement über wenigstens eine Kupplung miteinander verbunden sind und zumindest ein im implantierten Zustand an der Ankoppelstelle anliegender Abschnitt des Koppelelements zur verlustarmen Schwingungseinleitung in die Ankoppelstelle ausgelegt ist, wobei eine erste Kupplungshälfte der Kupplung eine Außenkontur mit mindestens näherungsweise der Gestalt einer Kugelkalotte aufweist, die in einer zur Außenkontur wenigstens teilweise komplementären Innenkontur einer zweiten Kupplungshälfte aufnehmbar ist, und wobei die Kupplung gegen Reibkräfte reversibel verschwenk- und/oder drehbar, jedoch bei im implantierten Zustand auftretenden dynamischen Kräften im Wesentlichen starr ist. Entsprechend einer abgewandelten Ausführungsform einer solchen Koppelanordnung (EP-Patentanmeldung 00 118 996.8) hat eine erste Kupplungshälfte der Kupplung eine Außenkontur mit mindestens näherungsweise zylindrischer, vorzugsweise kreiszylindrischer, Gestalt, die in einer zur Außenkontur wenigstens teilweise komplementären Innenkontur einer zweiten Kupplungshälfte aufnehmbar ist, wobei ein im implantierten Zustand an der Ankoppelstelle anliegender Abschnitt des Koppelelements zur verlustarmen Schwingungseinleitung in die Ankoppelstelle ausgelegt ist, wobei im implantierten Zustand eine Übertragung von dynamischen Kräften zwischen den beiden Kupplungshälften der Kupplung im Wesentlichen in Richtung der Längsachse der ersten Kupplungshälfte erfolgt, und wobei die Kupplung reversibel an- und abkuppelbar sowie reversibel linear und/oder rotatorisch mit Bezug auf eine Längsachse der ersten Kupplungshälfte verstellbar, jedoch bei im implantierten Zustand auftretenden dynamischen Kräften starr ist.

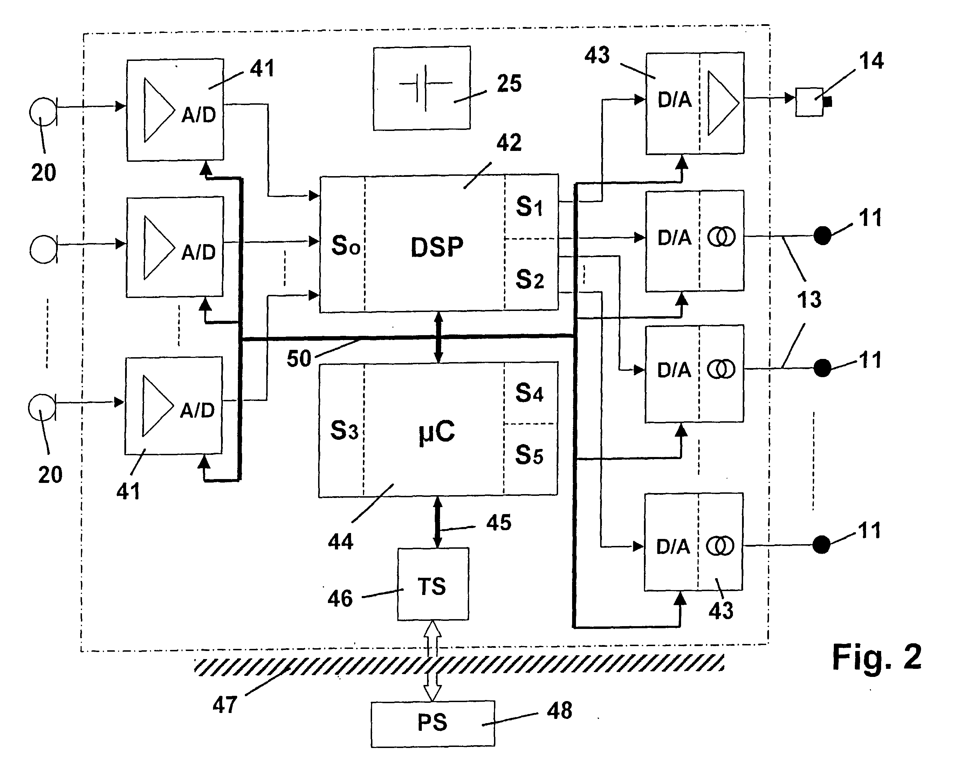

Zu dem in Fig. 1 dargestellten vollständig implantierbaren Hörsystem gehören ferner ein implantierbares Mikrofon 20, eine drahtlose Fernbedienung 21 zur Steuerung der Implantatfunktionen durch den Implantatträger sowie ein drahtloses, transkutanes Ladesystem mit einem Ladegerät 22 und einer Ladespule 23 zur Nachladung einer im Implantat befindlichen sekundären Batterie 25 (Fig. 2) zur Energieversorgung des Hörsystems.

Das Mikrofon 20 kann vorteilhaft in der aus EP-A-0 831 673 bekannten Weise aufgebaut und mit einer Mikrofonkapsel, die in einem Gehäuse allseitig hermetisch dicht untergebracht ist, sowie mit einer elektrischen Durchführungsanordnung zum Durchführen mindestens eines elektrischen Anschlusses von dem Innenraum des Gehäuses zu dessen Außenseite versehen sein, wobei das Gehäuse mindestens zwei Schenkel aufweist, die in einem Winkel mit Bezug aufeinander ausgerichtet sind, wobei der eine Schenkel die Mikrofonkapsel aufnimmt und mit einer Schalleintrittsmembran versehen ist, wobei der andere Schenkel die elektrische Durchführungsanordnung enthält und gegenüber der Ebene der Schalleintrittsmembran zurückversetzt ist, und wobei die Geometrie des Mikrofongehäuses so gewählt ist, dass bei Implantation des Mikrofons in der Mastoidhöhle der die Schalleintrittsmembran enthaltende Schenkel vom Mastoid aus in eine artifizielle Bohrung in der hinteren, knöchernen Gehörgangswand hineinragt und die Schalleintrittsmembran die Haut der Gehörgangswand berührt. Zur Festlegung des implantierten Mikrofons 20 kann zweckmäßig ein Fixationselement der aus US-A-5 999 632 bekannten Art vorgesehen sein, das eine Manschette aufweist, die mit einem zylindrischen Gehäuseteil den die Schalleintrittsmembran enthaltenden Schenkel umschließt und mit gegen die der Gehörgangshaut zugewendete Seite der Gehörgangswand anlegbaren, vorspringenden, elastischen Flanschteile versehen ist. Dabei beinhaltet das Fixationselement vorzugsweise eine Halterung, welche die genannten Flanschteile vor der Implantation entgegen einer elastischen Rückstellkraft der Flanschteile in einer das Durchstecken durch die Bohrung der Gehörgangswand erlaubenden umgebogenen Stellung hält.

Die an den Ausgang des Ladegerätes 22 angeschlossene Ladespule 23 bildet vorzugsweise in der aus US-A-5 279 292 bekannten Art Teil eines Sende-Serienresonanzkreises, der mit einem nicht veranschaulichten Empfangs-Serienresonanzkreis induktiv gekoppelt werden kann. Der Empfangs-Serienresonanzkreis kann Teil eines implantierbaren Elektronikmoduls 34 (Fig. 2) sein und entsprechend US-A-5 279 292 eine Konstantstromquelle für die Batterie 25 (Fig. 2) bilden. Dabei liegt der Empfangs-Serienresonanzkreis in einem Batterie-Ladestromkreis, der in Abhängigkeit von der jeweiligen Phase des in dem Ladestromkreis fließenden Ladestromes über den einen oder den anderen Zweig einer Vollweg-Gleichrichterbrücke geschlossen wird.

Das Elektronikmodul 34 ist bei der Anordnung nach Fig. 1 über eine Mikrofonleitung 35 an das Mikrofon 20, über eine Wandlerzuleitung 36 an den elektromechanischen Wandler 14 und über eine Array-Zuleitung 37 an das intracochleäre Reizelektrodenarray 10 angeschlossen.

Fig. 2 zeigt den möglichen Aufbau des signalverarbeitenden Elektronikmoduls 34 des mindestens teilweise implantierbaren Hörsystems. Über einen oder mehrere Schallsensoren (Mikrofone) 20 wird das externe Schallsignal aufgenommen und in elektrische Signale umgewandelt. Die analogen elektrischen Sensorsignale werden an Module 41 geleitet, wo sie vorverarbeitet, insbesondere vorverstärkt, und in digitale Signale umgewandelt (A/D) werden. Diese Vorverarbeitung kann beispielsweise in einer analogen linearen oder nicht-linearen Vorverstärkung und Filterung (zum Beispiel Antialiasing-Filterung) bestehen.

Die digitalisierten Sensorsignale werden in einem digitalen Signalprozessor 42 (DSP) weiterverarbeitet. Der Signalprozessor 42 enthält einen nicht überschreibbaren Festspeicherbereich S₀, in welchem die für einen "Minimalbetrieb" des Systems erforderlichen Anweisungen und Parameter gespeichert sind, sowie Speicherbereiche S₁ und S₂, in denen die Betriebssoftware der bestimmungsgemäßen Funktion beziehungsweise Funktionen des Implantatsystems abgelegt sind. Die wiederholt beschreibbaren Programmspeicher S₁ und S₂, zur Aufnahme der Betriebssoftware können auf EEPROM-Basis oder statischen RAM-Zellen basieren, wobei im letztgenannten Fall dafür gesorgt sollte, dass dieser RAM-Bereich immer durch das implantatinterne Energieversorgungssystem "gepuffert" ist.

Die digitalen Ausgangssignale des Signalprozessors 42 werden in Digital-Analog-Wandlern (D/A) 43 in Analogsignale umgewandelt und verstärkt sowie dann den Reizelektroden 11 und dem ausgangsseitigen elektromechanischen Wandler 14 zugeführt.

Der Signalprozessor 42 führt die bestimmungsgemäße Funktion des Hörimplantats aus. Dazu gehören eine Audiosignalverarbeitung für die Rehabilitation einer Hörstörung und gegebenenfalls auch eine Signalgenerierung im Fall eines Systems mit zusätzlicher Tinnitusmaskierer- oder Noiser-Funktion. Weiterhin enthält der digitale Signalprozessor 42 Softwaremodule, die eine duale Ansteuerung des Reizelektrodenarrays 10 und des elektromechanischen Wandlers 14 so vornehmen, dass die spektralen, zeitlichen, amplituden- und phasenbezogenen Wandler- beziehungsweise Reizelektroden-Signaleigenschaften so konfiguriert sind, dass bei dem betreffenden Patienten der optimale Hörerfolg erzielt wird. Diese Softwaremodule können statisch und dynamisch ausgelegt sein. Unter statischer Auslegung soll dabei verstanden werden, dass die Softwaremodule aufgrund wissenschaftlicher Erkenntnisse einmalig im Programmspeicher des Signalprozessors 42 abgelegt werden und unverändert bleiben.

Dynamisch bedeutet, dass diese Softwaremodule "lernfähig" sind, um dem angestrebten optimalen Hörerfolg zeitlich iterativ möglichst nahe zu kommen. Dies bedeutet, dass die Softwaremodule adaptiv ausgelegt sein können und die Parameteranpassung durch "Training" durch den Implantatträger und gegebenenfalls weitere Hilfsmittel, wie Rehabilitationsprogramme, vorgenommen wird. Weiterhin kann ein Softwaremodul enthalten sein, welches das Ziel der möglichst optimalen Hörversorgung auf der Basis eines lernfähigen neuronalen Netzwerkes approximiert. Das Training dieses neuronalen Netzes kann wieder durch den Implantatträger und/oder unter Zuhilfenahme weiterer externer Hilfsmittel erfolgen.

Damit die beschriebenen, software-basierten Algorithmen zur möglichst optimalen dualen Stimulation des geschädigten Gehörs insbesondere in einem Vollimplantat auch postoperativ implementiert werden können, enthält das System nach Fig. 2 einen weiteren Mikroprozessorbaustein, zum Beispiel einen Mikrocontroller (µC) 44 mit zugehörigen Speichern S₃, S₄ und S₅. Bei dem Speicher S₃ handelt es sich um einen wiederholt beschreibbaren Speicher, in welchem ein Arbeitsprogramm für den Microcontroller 44 abgelegt ist. In den Speicherbereichen S₄ und S₅ können insbesondere die Betriebsoftwareanteile des Implantatmanagementsystems abgelegt sein (zum Beispiel Verwaltungsüberwachungs- und Telemetriefunktionen). In den Speichern S₁ und/oder S₂, und/oder S₄ und/oder S₅ können auch von außen veränderliche, patientenspezifische wie zum Beispiel audiologische Anpassparameter, abgelegt sein.

Der Mikrocontroller 44 kommuniziert einerseits über einen bidirektionalen Datenbus 45 und ein Telemetriesystem (TS) 46 drahtlos (beispielsweise mittels induktiver Kopplung) durch die bei 47 angedeutete geschlossene Haut mit einem externen Programmiersystem (PS) 48. Das Programmiersystem 48 kann ein PC-basiertes System mit entsprechender Programmier-, Bearbeitungs-, Darstellungs- und Verwaltungssoftware sein. Über diese Telemetrieschnittstelle wird die zu verändernde beziehungsweise ganz auszutauschende Betriebssoftware des Implantatsystems übertragen und zunächst in dem Speicherbereich S₄ und/oder S₅ des Microcontrollers 44 zwischengespeichert. So kann zum Beispiel eine einfache Verifikation der Softwareübertragung durch einen Lesevorgang über die Telemetrieschnittstelle durchgeführt werden, bevor die Betriebssoftware oder die entsprechenden Signalverarbeitungsanteile dieser Software in die Programmspeicherbereiche S₁ und S₂, des digitalen Signalprozessors 42 über einen Datenbus 50 übertragen werden. Ferner kann auch das Arbeitsprogramm für den Microcontroller 44 über die Telemetrieschnittstelle ganz oder teilweise mit Hilfe der externen Einheit 48 geändert oder ausgetauscht werden.

Andererseits steuert der Mikrocontroller 44 implantatintern über den bidirektionalen Datenbus 50 die A/D-Wandler 41 der Sensor-Vorverarbeitung, die D/A-Wandler 43 zur Ansteuerung der Reizelektroden 11 und des elektromechanischen Wandlers 14 und den Signalprozessor 42 selbst. Die D/A-Wandler 43 können auch ganz oder teilweise wegfallen, wenn für die Reizelektroden digital gesteuerte Stromquellen vorgesehen sind und/oder wenn bei Verwendung eines elektromagnetischen Ausgangswandlers 14 ein zum Beispiel pulsweitenmoduliertes, serielles digitales Ausgangssignal des Signalprozessors 42 direkt an den Wandler 14 übermittelt wird. Über den Datenbus 50 werden auch Programmteile oder ganze Softwaremodule zwischen Außenwelt, Mikrokontroller 44 und Signalprozessor 42 übermittelt.

Das Implantatsystem enthält bei vollimplantierter Ausführungsform auch die primäre oder sekundäre Batteriezelle 25, die die einzelnen Module mit elektrischer Betriebsenergie versorgt und die zweckmäßig mit dem Elektronikmodul 34 zusammengefasst ist.

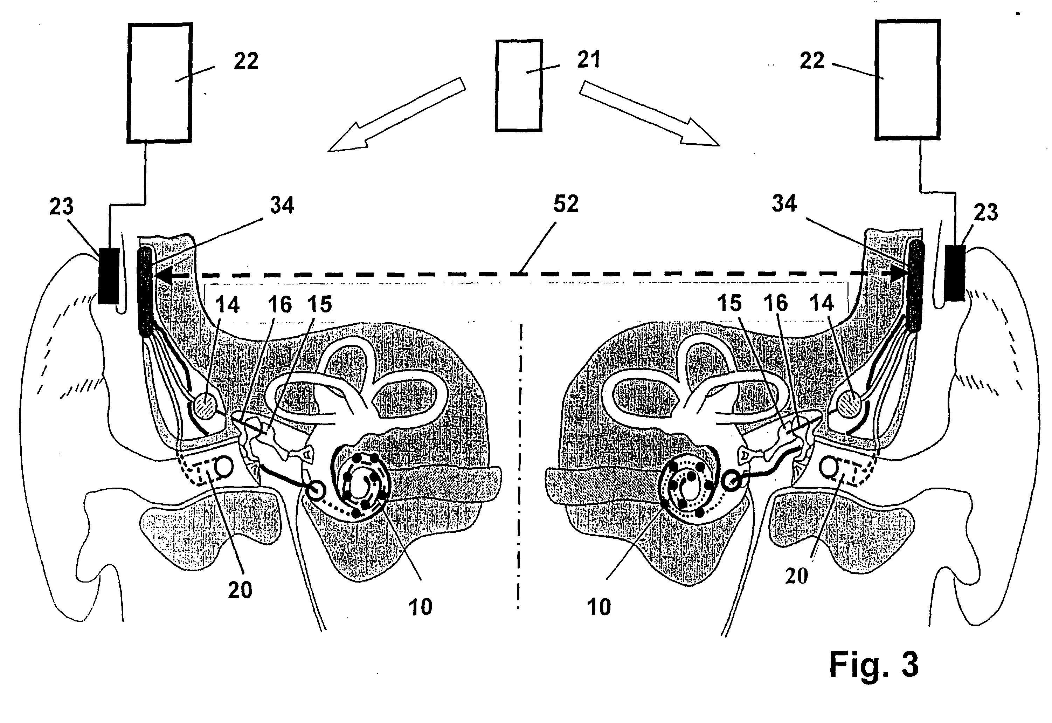

Fig. 3 zeigt eine binaurale Anwendung eines Hörimplantats gemäß Fig. 1. Dabei kommunizieren die Signalverarbeitungsmodule 34 über eine drahtgebundene implantierbare Leitungsverbindung 52 so miteinander, dass eine optimale binaurale Signalverarbeitung und Wandler- beziehungsweise Reizelektrodenarray-Ansteuerung in beiden implantierten Ohren erreicht wird. Weiterhin sind auch hier transkutane Ladevorrichtungen 22, 23 für den Fall implantatseitiger sekundärer Energiespeicherelemente (Batterien 25) vorgesehen sowie eine beide Elektronikmodule 34 synchron steuernde drahtlose Fernbedienung 21 zur Anwendung durch den Implantatträger.

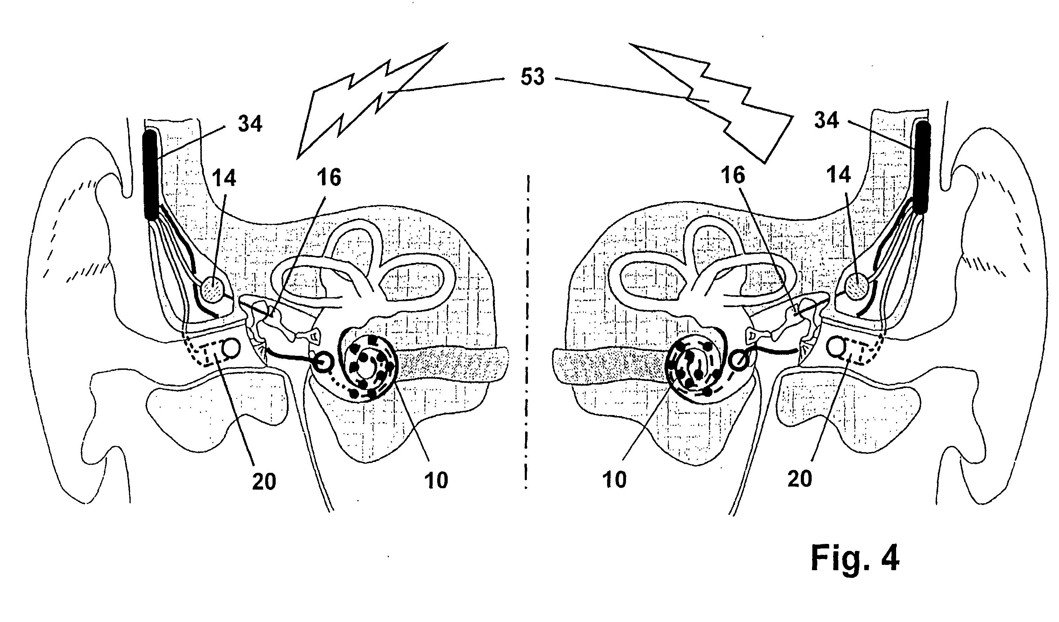

Fig. 4 zeigt die binaurale Anwendung eines Hörimplantats gemäß Fig. 1, bei dem die Signalverarbeitungsmodule 34 über eine drahtlose Verbindung (zum Beispiel eine bei 53 angedeutete bidirektionale Hochfrequenzstrecke) so miteinander kommunizieren, dass eine optimale binaurale Signalverarbeitung und Wandler- beziehungsweise Reizelektrodenarray-Ansteuerung in beiden implantierten Ohren erreicht wird. Weiterhin sind auch hier transkutane Ladevorrichtungen 22, 23 (nicht dargestellt) für den Fall implantatseitiger sekundärer Energiespeicherelemente (Batterien 25) vorgesehen sowie eine drahtlose Fernbedienung 21 zur Anwendung durch den Implantatträger, die beide Elektronikmodule 34 synchron steuert.

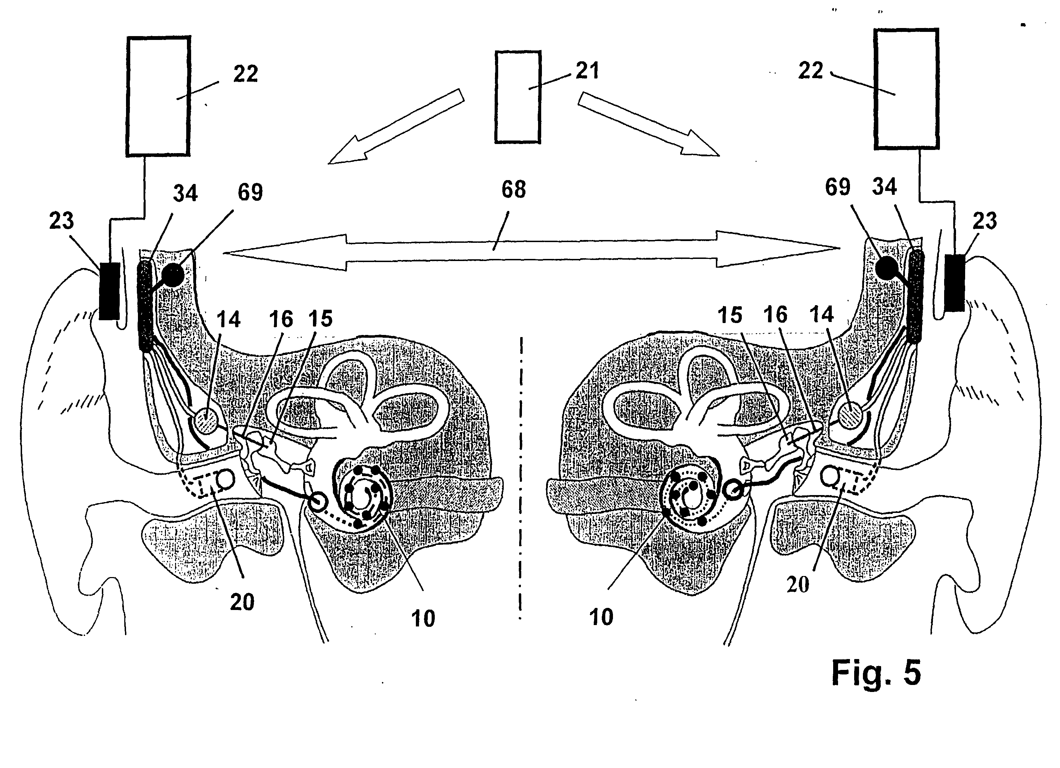

Die binaurale Ausbildung des Hörimplantats gemäß Fig. 5 unterscheidet sich von denjenigen der Fig. 4 nur dadurch, dass für die drahtlose Kommunikation zwischen den Signalverarbeitungsmodulen 34 beider Systemeinheiten eine körperschallgekoppelte Ultraschallstrecke 54 mit Ultraschall-Kopplern 55 vorgesehen ist. Dabei sind die zum Beispiel digitalen, bidirektionalen Informationen auf einen Träger im Ultraschallbereich vorzugsweise amplituden- oder frequenzmoduliert. Die Ultraschall-Koppler 55 können, wie in Fig. 5 dargestellt, von dem Elektronikmodul 34 örtlich getrennte und über elektrische Leitungen angeschlossene Ultraschallempfänger und -Sender sein, die vorzugsweise im Mastoidbereich an den Schädelknochen fest angekoppelt sind. Die Ultraschall-Koppler können aber auch in den Elektronikmodulen 34 integriert sein (nicht dargestellt), wenn die Elektronikmodule so im Mastoidbereich implantiert werden, dass eine Ultraschall-Körperübertragung durch den Schädelknochen stattfinden kann.

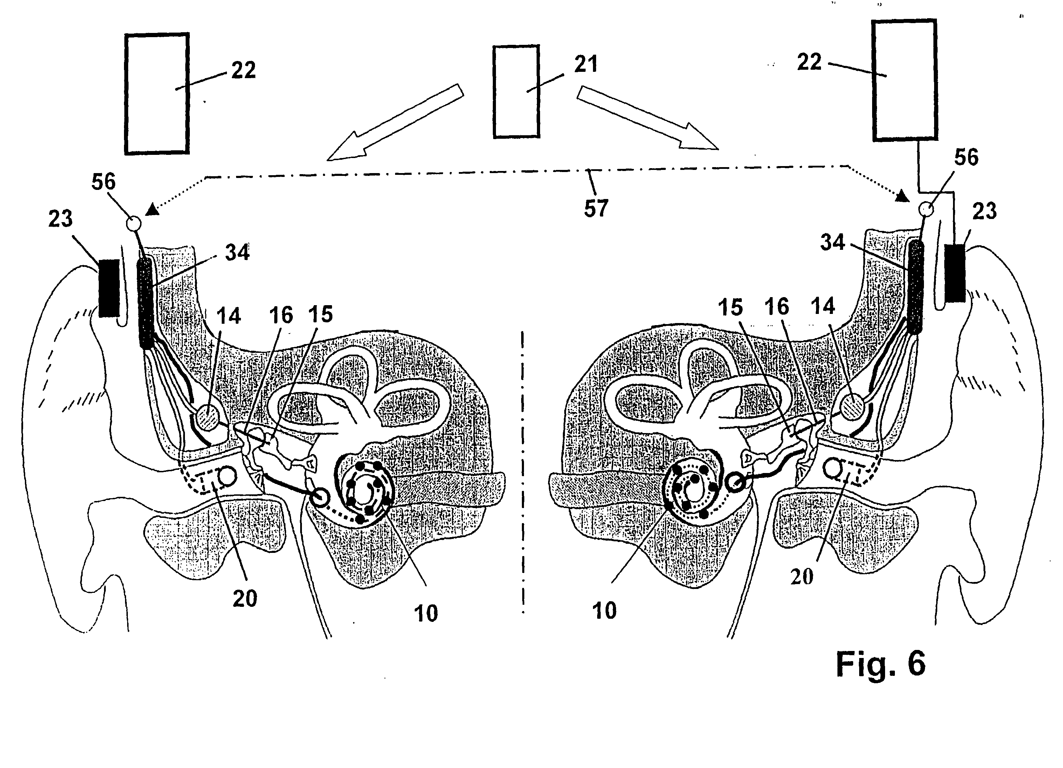

Eine weiter abgewandelte Ausführungsform eines binaural ausgebildeten Hörimplantats ist in Fig. 6 dargestellt. Bei dieser Ausführungsform werden abweichend von den Ausführungsbeispielen der Figuren 3 bis 5 die zum Beispiel digitalen, bidirektionalen Informationen implantatseitig auf einen Träger vorzugsweise amplituden- oder frequenzmoduliert und an implantierte Elektroden 56 angelegt, die Teil einer durch Körpergewebe des Implantatträgers führenden Datenübertragungsstrecke 57 sind. Es wird so ein modulierter Gewebestrom erhalten, der in an sich bekannter Weise (DE-A 38 31 809) für die gewünschte Kommunikation zwischen den Signalverarbeitungsmodulen 34 beider Systemeinheiten sorgt.

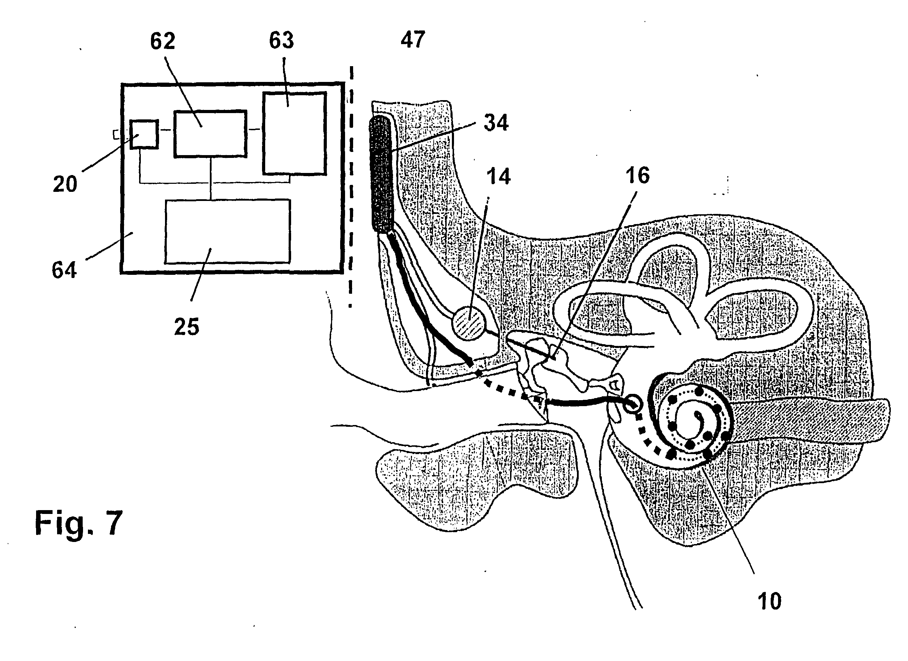

Fig. 7 zeigt schematisch den Aufbau eines monaural teilimplantierbaren Hörsystems mit intracochleären Reizelektrodenarray 10 und elektromechanischem Wandler 14 gemäß Fig. 1. Bei diesem teilimplantierbaren System sind ein Mikrofon 20, ein Elektronikmodul 62 für eine elektronische Signalverarbeitung weitestgehend entsprechend Fig. 2 (aber ohne das Telemetriesystem 46), die Energieversorgung (Batterie) 25 sowie eine Modulator/Sender-Einheit 63 in einem extern am Körper, vorzugsweise am Kopf über dem Implantat, zu tragenden externen Modul 64 enthalten. Das Implantat ist wie bei bekannten Teilimplantaten energetisch passiv. Sein Elektronikmodul 34 (ohne Batterie 52) empfängt Betriebsenergie und Wandler- beziehungsweise Reizelektroden-Steuerdaten 10 über die Modulator/Sender-Einheit 63 im externen Teil 64.

Es versteht sich, dass auch ein teilimplantierbares System binaural appliziert werden kann und dass dann für eine Kommunikation zwischen den beiden Systemeinheiten vorzugsweise entsprechend den in den Figuren 3 bis 6 veranschaulichen Ausführungsbeispielen von binauralen Anwendungen vollimplantierbarer Systeme gesorgt werden kann.

## Patentansprüche

1. Mindestens teilweise implantierbares System zur Rehabilitation einer Hörstörung, das mindestens einen Sensor (20) zur Aufnahme des Schallsignals und dessen Umwandlung in entsprechende elektrische Signale, eine elektronische Signalverarbeitungseinheit (34, 34', 62) zur Audiosignalverarbeitung und -verstärkung, eine elektrische Energieversorgungseinheit (25), die einzelne Komponenten des Systems mit Strom versorgt, sowie eine ausgangsseitige aktorische Stimulationsanordnung (10, 14) umfasst, wobei die ausgangsseitige Stimulationsanordnung in Kombination einen elektromechanischen Wandler (14) zur mechanischen Stimulation des Mittel- oder Innenohres und ein intracochleäres, elektrisch wirkendes Reizelektrodenarray (10) mit mindestens einer Reizelektrode (11) zur elektrischen Stimulation des Innenohres aufweist, **dadurch gekennzeichnet, dass** die Signalverarbeitungseinheit (34, 34', 62) einen digitalen Signalprozessor (42) aufweist, der Softwaremodule enthält, die die Ansteuerung des elektromechanischen Wandlers (14) und des Reizelektrodenarrays (10) so vornehmen, dass die spektralen, zeitlichen, amplituden- und phasenbezogenen Wandler- beziehungsweise Reizelektroden-Signaleigenschaften so konfiguriert sind, dass patientenspezifisch ein optimaler Hörerfolg erzielt wird.

2. System nach Anspruch 1, **dadurch gekennzeichnet, dass** der ausgangsseitige elektromechanische Wandler (14) als elektromagnetischer, elektrodynamischer, piezoelektrischer, magnetostriktiver oder dielektrischer (kapazitiver) Wandler ausgebildet ist

3. System nach Anspruch 1 oder 2, **dadurch gekennzeichnet, dass** der ausgangsseitige elektromechanische Wandler (14) für eine direkte mechanische Kopplung mit dem Mittelohr oder für eine berührungslose, luftspaltgekoppelte Ankopplung an das Mittelohr nach dem elektromagnetischen Wandlerprinzip ausgelegt ist.

4. System nach Anspruch 1 oder 2, **dadurch gekennzeichnet, dass** der ausgangsseitige elektromechanische Wandler (14) für eine direkte hydromechanische Kopplung mit dem Innenohr ausgelegt ist.

5. System nach einem der vorhergehenden Ansprüche, **dadurch gekennzeichnet, dass** der ausgangsseitige elektromechanische Wandler (14) einen Übertragungsbereich von etwa 100 Hz bis etwa 10 kHz hat.

6. System nach einem der vorhergehenden Ansprüche, **dadurch gekennzeichnet, dass** der ausgangsseitige elektromechanische Wandler (14) so abgestimmt ist, dass seine erste mechanische Resonsanzfrequenz am oberen Ende des angestrebten Übertragungsfrequenzbereiches, insbesondere bei etwa 8 kHz bis etwa 10 kHz, liegt.

7. System nach einem der vorhergehenden Ansprüche, **dadurch gekennzeichnet, dass** der ausgangsseitige elektromechanische Wandler (14) hermetisch dicht aufgebaut ist.

8. System nach einem der vorhergehenden Ansprüche, **dadurch gekennzeichnet, dass** die Signalverarbeitungseinheit (34, 34', 62) eine Vorverarbeitungsanordnung (41) zum Vorverstärken und/oder Filtern sowie zum Analog-Digital- (A/D-)Wandeln der Schallsensorsignale aufweist.

9. System nach Anspruch 8, **dadurch gekennzeichnet, dass** bei Vorhandensein mehrerer Schallsensoren (20) jedem der Schallsensoren ein Analog-Digital Wandler (41) nachgeschaltet ist.

10. System nach einem der vorhergehenden Ansprüche, **dadurch gekennzeichnet, dass** die Signalverarbeitungseinheit (34, 34', 62) Softwaremodule enthält, die parallel zum Hörgerätebetrieb die Maskierung eines Tinnitus ermöglichen.

11. System nach einem der vorhergehenden Ansprüche, **dadurch gekennzeichnet, dass** die Signalverarbeitungseinheit (34, 34', 62) einen digitalen Signalprozessor (42) zum Verarbeiten der A/D-gewandelten und gegebenenfalls mittels der Vorverarbeitungsanordnung (41) vorverarbeiteten Schallsensorsignale und/oder zum Generieren von digitalen Signalen für eine Tinnitusmaskierung aufweist.

12. System nach einem der vorhergehenden Ansprüche, **gekennzeichnet durch** mindestens einen der ausgangsseitigen Stimulationsanordnung (10, 14) vorgeschalteten Digital-Analog-Wandler (43).

13. System nach Anspruch 12, **dadurch gekennzeichnet, dass** dem ausgangsseitigen elektromechanischen Wandler (14) und der oder den Elektrode(n) (11) des Reizelektrodenarrays (10) jeweils ein eigener Digital-Analog-Wandler (43) vorgeschaltet ist.

14. System nach einem der vorhergehenden Ansprüche, **dadurch gekennzeichnet, dass** die Softwaremodule statisch in der Weise ausgelegt sind, dass sie aufgrund wissenschaftlicher Erkenntnisse einmalig in einem Programmspeicher (S₁, S₂,) des digitalen Signalprozessors (42) abgelegt werden und unverändert bleiben.

15. System nach einem der vorhergehenden Ansprüche, **gekennzeichnet durch** eine vorzugsweise PC-basierte, drahtlose Telemetrieeinrichtung (46) zur Übertragung von Daten zwischen dem implantierten Teil (34) des Systems und einer externen Einheit, insbesondere einem externen Programmiersystem (48).

16. System nach Anspruch 15, **dadurch gekennzeichnet, dass** die Softwaremodule dynamisch (lernfähig) ausgelegt sind.

17. System nach Anspruch16, **dadurch gekennzeichnet, dass** die Softwaremodule für eine Parameteranpassung durch "Training" durch den Implantatträger und/oder unter Zuhilfenahme weiterer externer Hilfsmittel adaptiv ausgelegt sind.

18. System nach einem der vorhergehenden Ansprüche, **dadurch gekennzeichnet, dass** der digitale Signalprozessor ein Softwaremodul zum Approximieren einer möglichst optimalen Stimulation auf der Basis eines lernfähigen neuronalen Netzwerkes enthält.

19. System nach Anspruch 18, **dadurch gekennzeichnet, dass** das neuronale Netzwerk für ein "Training" durch den Implantatträger und/oder für ein Lernen unter Zuhilfenahme externer Hilfsmittel ausgelegt ist.

20. System nach einem der Ansprüche 11 bis 19, **dadurch gekennzeichnet, dass** dem Signalprozessor (42) zur Aufnahme und Wiedergabe eines Betriebsprogramms eine wiederholt beschreibbare, implantierbare Speicheranordnung (S₁, S₂,) zugeordnet ist, und mindestens Teile des Betriebsprogramms durch von der externen Einheit (48) über die Telemetrieeinrichtung (46) übermittelte Daten geändert oder ausgetauscht werden können.

21. System nach Anspruch 20, **dadurch gekennzeichnet, dass** ferner eine Zwischenspeicheranordnung (S₄, S₅) vorgesehen ist, in welcher von der externen Einheit (48) über die Telemetrieeinrichtung (46) übermittelte Daten vor dem Weiterleiten an den Signalprozessor (42) zwischengespeichert werden können.

22. System nach Anspruch 21, **dadurch gekennzeichnet, dass** ferner eine Überprüfungslogik (44) vorgesehen ist, um in der Zwischenspeicheranordnung (S₄, S₅) gespeicherte Daten vor dem Weiterleiten an den Signalprozessor (42) einer Überprüfung zu unterziehen.

23. System nach einem der Ansprüche 11 bis 22, **gekennzeichnet durch** einen Mikroprozessorbaustein (44), insbesondere einen Microcontroller, zum implantatinternen Steuern der A/D-Wandler (41) und/oder der D/A-Wandler (43) und/oder des Signalprozessors (42) über einen Datenbus (50).

24. System nach den Ansprüchen 22 und 23, **dadurch gekennzeichnet, dass** die Überprüfungslogik und die Zwischenspeicheranordnung (S₄, S₅) in dem Mikroprozessorbaustein (44) implementiert sind.

25. System nach Anspruch 23 oder 24, **dadurch gekennzeichnet, dass** über den Datenbus (50) und die Telemetrieeinrichtung (46) auch Programmteile oder ganze Softwaremodule zwischen der Außenwelt, dem Mikroprozessorbaustein (44) und dem Signalprozessor (42) übermittelbar sind.

26. System nach einem der Ansprüche 23 bis 25, **dadurch gekennzeichnet, dass** dem Mikroprozessorbaustein (44) eine implantierbare Speicheranordnung (S₃) zum Speichern eines Arbeitsprogramms für den Mikroprozessorbaustein zugeordnet ist, und mindestens Teile des Arbeitsprogramms für den Mikroprozessorbaustein durch von der externen Einheit (48) über die Telemetrieeinrichtung (46) übermittelte Daten geändert oder ausgetauscht werden können.

27. System nach einem der Ansprüche 21 bis 26, **dadurch gekennzeichnet, dass** mindestens zwei Speicherbereiche (S₁, S₂,) zur Aufnahme und Wiedergabe mindestens des Betriebsprogramms des Signalprozessors (42) vorgesehen sind.

28. System nach einem der Ansprüche 22 bis 27, **dadurch gekennzeichnet, dass** die Zwischenspeicheranordnung mindestens zwei Speicherbereiche (S₄, S₅) zur Aufnahme und Wiedergabe von von der externen Einheit (48) über die Telemetrieeinrichtung (46) übermittelten Daten aufweist.

29. System nach einem der Ansprüche 11 bis 28, **dadurch gekennzeichnet, dass** dem Signalprozessor (42) ferner ein vorprogrammierter, nicht überschreibbarer Festspeicherbereich (S₀) zugeordnet ist.

30. System nach einem der Ansprüche 11 bis 29, **dadurch gekennzeichnet, dass** die Telemetrieeinrichtung (46) zur Übermittlung auch von Betriebsparametern zwischen dem implantierbaren Teil (34) des Systems und der externen Einheit (48) ausgelegt ist.

31. System nach einem der vorhergehenden Ansprüche, **dadurch gekennzeichnet, dass** es vollimplantierbar ausgebildet und mit mindestens einem implantierbaren Schallsensor (20) versehen ist, die elektrische Energieversorgungseinheit implantatseitig ein nachladbares elektrisches Speicherelement (25) aufweist und eine drahtlose, transkutane Ladevorrichtung (22, 23) zum Laden des Speicherelements vorgesehen ist.

32. System nach Anspruch 31, **gekennzeichnet durch** eine drahtlose Fernbedienung (21) zur Steuerung der Implantatfunktionen **durch** den Implantatträger.

33. System nach einem der Ansprüche 1 bis 30, **dadurch gekennzeichnet, dass** es teilimplantierbar ausgebildet ist, wobei mindestens ein Schallsensor (20), die elektronische Signalverarbeitungseinheit (62), die Energieversorgungseinheit (52) sowie eine Modulator/Sender-Einheit (63) in einem extern am Körper, vorzugsweise am Kopf über dem Implantat (34'), zu tragenden externen Modul (64) enthalten sind, sowie das Implantat energetisch passiv ist und seine Betriebsenergie und Wandlersteuerdaten über die Modulator/Sender-Einheit im externen Modul empfängt.

34. System nach einem der vorhergehenden Ansprüche, **dadurch gekennzeichnet, dass** es als binaurales System zur Rehabilitation einer Hörstörung beider Ohren ausgelegt ist, das zwei Systemeinheiten aufweist, die jeweils einem der beiden Ohren zugeordnet sind.

35. System nach Anspruch 34, **dadurch gekennzeichnet, dass** die beiden Systemeinheiten einander im wesentlichen gleich sind.

36. System nach Anspruch 34, **dadurch gekennzeichnet, dass** die eine Systemeinheit als Master-Einheit und die andere Systemeinheit als von der Master-Einheit gesteuerte Slave-Einheit ausgelegt ist.

37. System nach einem der Ansprüche 34 bis 36, **gekennzeichnet durch** eine drahtgebundene implantierbare Leitungsverbindung (52), über welche die Signalverarbeitungsmodule (34) so miteinander kommunizieren, dass in beiden Systemeinheiten eine optimierte binaurale Signalverarbeitung und Wandler-Array-Ansteuerung erreicht wird.

38. System nach einem der Ansprüche 34 bis 36, **gekennzeichnet durch** eine drahtlose Verbindung (53), vorzugsweise eine bidirektionale Hochfrequenzstrecke, über welche die Signalverarbeitungsmodule (34) so miteinander kommunizieren, dass in beiden Systemeinheiten eine optimierte binaurale Signalverarbeitung und Wandler-Array-Ansteuerung erreicht wird.

39. System nach einem der Ansprüche 34 bis 36, **dadurch gekennzeichnet, dass** die Signalverarbeitungsmodule (34) unter Verwendung von Ultraschall-Kopplern (55) über eine körperschallgekoppelte Ultraschallstrecke (54) so miteinander kommunizieren, dass in beiden Systemeinheiten eine optimierte binaurale Signalverarbeitung und Wandler-Array-Ansteuerung erreicht wird.

40. System nach einem der Ansprüche 34 bis 36, **dadurch gekennzeichnet, dass** den Signalverarbeitungsmodulen (34) implantierbare Elektroden (56) zugeordnet sind, die im implantierten Zustand Teil einer durch Körpergewebe des Implantatträgers führenden Datenübertragungsstrecke (57) für eine Kommunikation der Signalverarbeitungsmodule (34) der beiden Systemeinheiten sind.

## Claims

1. At least partially implantable system for rehabilitation of a hearing disorder which comprises at least one sensor (20) for picking up the acoustic signal and converting it into corresponding electrical signals, an electronic signal processing unit (34, 34', 62) for audio signal processing and amplification, an electrical power supply unit (25) which supplies individual components of the system with energy, and an output-side actory stimulation arrangement (10, 14), wherein the output-side stimulation arrangement in combination has an electromechanical transducer (14) for mechanical stimulation of the middle or inner ear and an intracochlear, electrically acting stimulation electrode array (10) with at least one stimulation electrode (11) for electrical stimulation of the inner ear, **characterized in that** the signal processing unit (34, 34', 62) comprises a digital signal processor (42) which contains software modules which control the electromechanical transducer (14) and the stimulation electrode array (10), such that the spectral-, time-, amplitude- and phase-referenced transducer and stimulation electrode signal properties are configured such that optimum hearing success is achieved in a patient-specific manner.

2. System as claimed in claim 1, **characterized in that** the output-side electromechanical transducer (14) is made as electromagnetic, electrodynamic, piezoelectric, magnetostrictive or dielectric (capacitive) transducer.

3. System as claimed in claim 1 or 2, **characterized in that** the output-side electromechanical transducer (14) is designed for direct mechanical coupling to the middle ear or for contactless, air gap-coupled coupling to the middle ear according to the electromagnetic transducer principle.

4. System as claimed in claim 1 or 2, **characterized in that** the output-side electromechanical transducer (14) is designed for direct hydromechanical coupling to the inner ear.

5. System as claimed in any one of the preceding claims, **characterized in that** the output-side electromechanical transducer (14) has a transmission range from about 100 Hz to about 10 kHz.

6. System as claimed in any one of the preceding claims, **characterized in that** the output-side electromechanical transducer (14) is tuned such that its first mechanical resonant frequency is at the upper end of the desired transmission frequency range, especially at about 8 kHz about 10 kHz.

7. System as claimed in any one of the preceding claims, **characterized in that** the output-side electromechanical transducer (14) is built hermetically sealed.

8. System as claimed in any one of the preceding claims, **characterized in that** the signal processing unit (34, 34', 62) has a preprocessing arrangement (41) for preamplification and/or filtering and for analog-digital (A/D) conversion of the acoustic sensor signals.

9. System as claimed in claim 8, **characterized in that** in the presence of several acoustic sensors (20), an analog-digital converter (41) is connected to the output of each of the acoustic sensors.

10. System as claimed in any one of the preceding claims, **characterized in that** the signal processing unit (34, 34', 62) contains software modules which enable the masking of tinnitus parallel to the hearing aid function.

11. System as claimed in any one of the preceding claims, **characterized in that** the signal processing unit (34, 34', 62) has a digital signal processor (42) for processing the A/D-converted acoustic sensor signals which optionally have been preprocessed by means of the preprocessing arrangement (41) and/or for generation of digital signals for tinnitus masking.

12. System as claimed in any one of the preceding claims, **characterized by** at least one digital-analog converter (43) which is connected upstream of the output-side stimulation arrangement (10, 14).

13. System as claimed in claim 12, **characterized in that** the output-side electromechanical transducer (14) and the electrode(s) (11) of the stimulation electrode array (10) each have an own digital-analog converter (43) connected upstream thereof.

14. System as claimed in any one of the preceding claims, **characterized in that** the software modules are designed to be static such that as a result of scientific findings they are stored once in the program storage (S₁, S₂) of the digital signal processor (42) and remain unchanged.

15. System as claimed in any one of the preceding claims, **characterized by** a preferably PC-based wireless telemetry means (46) for transmission of data between the implanted part (34) of the system and an external unit, especially an external programming system (48).

16. System as claimed in claim 15, **characterized in that** the software modules are designed to be dynamic (adaptive).

17. System as claimed in claim 16, **characterized in that** the software modules are adaptively designed for parameter matching by "training" by the implant wearer and/or using further external aids.

18. System as claimed in any one of the preceding claims, **characterized in that** the digital signal processor contains a software module for approximation of a stimulation as optimum as possible based on an adaptive neural network.

19. System as claimed in claim 18, **characterized in that** the neural network is designed for "training" by the implant wearer and/or for learning using external aids.

20. System as claimed in any one of claims 11 to 19, **characterized in that** a rewritable implantable storage arrangement (S₁, S₂) for storage and retrieval of an operating program is assigned to the signal processor (42), and **in that** at least parts of the operating program can be replaced or changed by data transmitted from the external unit (48) via the telemetry means (46).

21. System as claimed in claim 20, **characterized by** further comprising a buffer storage arrangement (S₄, S₅) in which data transmitted from the external unit (48) via the telemetry means (46) can be buffered before they are relayed to the signal processor (42).

22. System as claimed in claim 21, **characterized by** further comprising a checking logic (44) for checking data stored in the buffer storage arrangement (S₄, S₅) before relaying the data to the signal processor (42).

23. System as claimed in any one of claims 11 to 22, **characterized by** a microprocessor module (44), especially a microcontroller, for control of the A/D converter (41) and/or the D/A converter (43) and/or the signal processor (42) within the implant via a data bus (50).

24. System as claimed in claims 22 and 23, **characterized in that** the checking logic and the buffer storage arrangement (S₄, S₅) are implemented in the microprocessor module (44).

25. System as claimed in claim 23 or 24, **characterized in that** via the data bus (50) and the telemetry means (46) also program parts or entire software modules can be transferred between the outside world, the microprocessor module (44) and the signal processor (42).

26. System as claimed in any one of claims 23 to 25, **characterized in that** an implantable storage arrangement (S₃) for storing a working program for the microprocessor module is assigned to the microprocessor module (44), and at least parts of the working program for the microprocessor module can be changed or replaced by data transferred from the external unit (48) via the telemetry means (46).

27. System as claimed in any one of claims 21 to 26, comprising at least two storage areas (S₁, S₂) for storage and retrieval of at least the operating program of the signal processor (42).

28. System as claimed in any one of claims 22 to 27, **characterized in that** the buffer storage arrangement comprises at least two storage areas (S₄, S₅) for storage and retrieval of data transferred from the external unit (48) via the telemetry means (46).

29. System as claimed in any one of claims 11 to 28, **characterized in that** a preprogrammed read-only memory area (S₀) which cannot be overwritten is assigned to the signal processor (42).

30. System as claimed in any one of claims 11 to 29, **characterized in that** the telemetry means (46) is designed for transmission also of operating parameters between the implanted part (34) of the system and the external unit (48).

31. System as claimed in any one of the preceding claims, **characterized in that** it is designed totally implantable and is provided with at least one implantable acoustic sensor (20), wherein the electrical power supply unit on the implant side comprises a rechargeable electrical storage element (25), and wherein there is provided a wireless, transcutaneous charging device (22, 23) for charging of the storage element.

32. System as claimed in claim 31, **characterized by** a wireless remote control (21) for control of the implant functions by the implant wearer.

33. System as claimed in any one of claims 1 to 30, **characterized in that** it is designed partially implantable, wherein at least one acoustic sensor (20), the electronic signal processing arrangement (62), the power supply unit (52) and a modulator/transmitter unit (63) are contained in an external module (64) which can be worn externally on the body, preferably on the head over the implant (34'), and wherein the implant is passive in terms of energy and receives its operating energy and transducer control data via the modulator/transmitter unit in the external module.

34. System as claimed in any one of the preceding claims, **characterized in that** it is designed as a binaural system for rehabilitation of a hearing disorder of both ears, which has two system units which each are assigned to one of the two ears.

35. System as claimed in claim 34, **characterized in that** the two system units are essentially identical to one another.

36. System as claimed in claim 34, **characterized in that** the one system unit is designed as master unit and the other system unit is designed as slave unit which is controlled by the master unit.

37. System as claimed in any one of claims 34 to 36, **characterized by** a wired implantable line connection (52) via which the signal processing modules (34) communicate with one another such that in both system units optimized binaural signal processing and transducer array control is achieved.

38. System as claimed in any one of claims 34 to 36, **characterized by** a wireless connection (53), preferably a bidirectional high frequency path, via which the signal processing modules (34) communicate with one another such that in both system units optimized binaural signal processing and transducer array control is achieved.

39. System as claimed in any one of claims 34 to 36, **characterized in that** the signal processing modules (34) communicate with one another by the use of ultrasonic couplers (55) via an ultrasonic path (54) coupled by bone conduction, such that in both system units optimized binaural signal processing and transducer array control is achieved.

40. System as claimed in any one of claims 34 to 36, **characterized in that** implantable electrodes (56) are assigned to the signal processing modules (34) and in the implanted state are part of a data transmission path (57) which leads through the body tissue of the implant wearer for communication of the signal processing modules (34) of the two system units.

## Revendications

1. Système au moins partiellement implantable destiné à corriger un trouble auditif, lequel comprend au moins : un capteur (20) servant à recevoir le signal acoustique et à le convertir en signaux électriques correspondants ; une unité électronique de traitement de signal (34, 34', 62) servant à traiter et à amplifier le signal audio ; une unité d'alimentation en énergie électrique (25) qui alimente différents composants du système en courant ; et un dispositif de stimulation actionneur (10, 14) situé côté sortie ; le dispositif de stimulation situé côté sortie comportant, de manière combinée, un transducteur électromécanique (14) assurant la stimulation mécanique de l'oreille moyenne ou de l'oreille interne et un groupement d'électrodes de stimulation (10) intracochléaire agissant électriquement et comptant au moins une électrode de stimulation (11) qui assure la stimulation électrique de l'oreille interne, **caractérisé en ce que** l'unité de traitement de signal (34, 34', 62) comporte un organe de traitement de signal numérique (42) qui contient des modules logiciels assurant la commande du transducteur électromécanique (14) et du groupement d'électrodes de stimulation (10) de telle sorte que les propriétés de signal - spectrales, temporelles, relatives à l'amplitude et à la phase - du transducteur et des électrodes de stimulation soient configurées de manière à obtenir, spécifiquement pour chaque patient, un résultat auditif optimal.

2. Système selon la revendication 1, **caractérisé en ce que** le transducteur électromécanique (14) situé côté sortie est réalisé sous forme de transducteur électromagnétique, électrodynamique, piézoélectrique, magnétostrictif ou diélectrique (capacitif).

3. Système selon la revendication 1 ou 2, **caractérisé en ce que** le transducteur électromécanique (14) situé côté sortie est conçu pour un couplage mécanique direct à l'oreille moyenne ou pour un couplage sans contact, par l'entrefer, à l'oreille moyenne, selon le principe du transducteur électromagnétique.

4. Système selon la revendication 1 ou 2, **caractérisé en ce que** le transducteur électromécanique (14) situé côté sortie est conçu pour un couplage hydromécanique direct à l'oreille interne.

5. Système selon l'une des revendications précédentes, **caractérisé en ce que** le transducteur électromécanique (14) situé côté sortie a un domaine de transmission d'environ 100 Hz jusqu'environ 10 kHz.

6. Système selon l'une des revendications précédentes, **caractérisé en ce que** le transducteur électromécanique (14) situé côté sortie est réglé de sorte que sa première fréquence de résonance mécanique soit située à l'extrémité supérieure du domaine de fréquences de transmission recherché, notamment dans une zone allant d'environ 8 kHz à environ 10 kHz.

7. Système selon l'une des revendications précédentes, **caractérisé en ce que** le transducteur électromécanique (14) situé côté sortie est conçu pour être hermétique.

8. Système selon l'une des revendications précédentes, **caractérisé en ce que** l'unité de traitement de signal (34, 34', 62) comporte un dispositif de prétraitement (41) servant à la préamplification et/ou au filtrage ainsi qu'à la conversion analogique-numérique (A/N) des signaux de capteur acoustique.

9. Système selon la revendication 8, **caractérisé en ce que**, s'il y a plusieurs capteurs acoustiques (20), un transducteur analogique-numérique (41) est placé en aval de chacun des capteurs acoustiques.

10. Système selon l'une des revendications précédentes, **caractérisé en ce que** l'unité de traitement de signal (34, 34', 62) contient des modules logiciels qui permettent le masquage d'un acouphène parallèlement au fonctionnement en prothèse auditive.

11. Système selon l'une des revendications précédentes, **caractérisé en ce que** l'unité de traitement de signal (34, 34', 62) comporte un organe de traitement de signal numérique (42) servant à traiter les signaux de capteur acoustique convertis du mode analogique au mode numérique, et le cas échéant prétraités au moyen du dispositif de prétraitement (41), et/ou à produire des signaux numériques destinés à masquer un acouphène.

12. Système selon l'une des revendications précédentes, **caractérisé par** au moins un transducteur analogique-numérique (43) placé en amont du dispositif de stimulation (10, 14) situé côté sortie.

13. Système selon la revendication 12, **caractérisé en ce que**, en amont du transducteur électromécanique (14) situé côté sortie et de l'électrode ou des électrodes (11) du groupement d'électrodes de stimulation (10), on place chaque fois un transducteur analogique-numérique (43) en propre.

14. Système selon l'une des revendications précédentes, **caractérisé en ce que** les modules logiciels sont statiques en ce sens qu'ils sont placés une fois pour toutes dans une mémoire de programme (S_{1'} S₂) de l'organe de traitement de signal numérique (42), sur la base de connaissances scientifiques, et qu'on ne les y modifie pas.

15. Système selon l'une des revendications précédentes, **caractérisé par** un dispositif télémétrique sans fil (46), de préférence installé sur ordinateur individuel (PC), servant à transmettre des données entre la partie implantée (34) du système et une unité extérieure, notamment un système de programmation extérieur (48).

16. Système selon la revendication 15, **caractérisé en ce que** les modules logiciels sont dynamiques (c'est-à-dire dotés d'une capacité d'apprentissage).

17. Système selon la revendication 16, **caractérisé en ce que** les modules logiciels sont adaptatifs de façon que des paramètres puissent être adaptés dans le cadre d'un "entraînement" effectué par le porteur d'implant et/ou en utilisant d'autres moyens extérieurs.

18. Système selon l'une des revendications précédentes, **caractérisé en ce que** l'organe de traitement de signal numérique contient un module logiciel servant à s'approcher autant que possible d'une stimulation optimale sur la base d'un réseau neuronal doté de capacités d'apprentissage.

19. Système selon la revendication 18, **caractérisé en ce que** le réseau neuronal est conçu pour que le porteur d'implant "s'entraîne" et/ou pour effectuer un apprentissage en utilisant des moyens extérieurs.

20. Système selon l'une des revendications 11 à 19, **caractérisé en ce qu'**un dispositif formant mémoire (S₁, S₂) qui est implantable et dans lequel on peut réécrire est associé à l'organe de traitement de signal (42) et sert à enregistrer et à restituer un programme d'exploitation, et **en ce qu'**au moins des parties du programme d'exploitation peuvent être modifiées ou remplacées par des données transmises par l'unité extérieure (48) par l'intermédiaire du dispositif télémétrique (46).

21. Système selon la revendication 20, **caractérisé en ce qu'**il est de plus prévu un dispositif formant mémoire intermédiaire (S₄, S₅) dans lequel des données transmises par l'unité extérieure (48) par l'intermédiaire du dispositif télémétrique (46) peuvent être mises en mémoire intermédiaire avant transmission à l'organe de traitement de signal (42).

22. Système selon la revendication 21, **caractérisé en ce qu'**il est de plus prévu une logique de contrôle (44) en vue de soumettre des données stockées dans le dispositif formant mémoire intermédiaire (S₄, S₅) à un contrôle avant transmission à l'organe de traitement de signal (42).

23. Système selon l'une des revendications 11 à 22, **caractérisé par** un module microprocesseur (44), notamment un microrégisseur, servant à commander, au sein de l'implant, les transducteurs analogiques-numériques (41) et/ou les transducteurs numériques-analogiques (43) et/ou l'organe de traitement de signal (42) par l'intermédiaire d'un bus de données (50).

24. Système selon les revendications 22 et 23, **caractérisé en ce que** la logique de contrôle et le dispositif formant mémoire intermédiaire (S₄, S₅) sont réalisés dans le module microprocesseur (44).

25. Système selon la revendication 23 ou 24, **caractérisé en ce qu'**il est également possible de transmettre des parties de programme ou des modules logiciels complets entre le monde extérieur, le module microprocesseur (44) et l'organe de traitement de signal (42) par l'intermédiaire du bus de données (50) et du dispositif télémétrique (46).

26. Système selon l'une des revendications 23 à 25, **caractérisé en ce qu'**un dispositif formant mémoire (S₃), implantable et servant à stocker un programme de travail destiné au module microprocesseur, est associé au module microprocesseur (44), et **en ce qu'**au moins des parties du programme de travail destiné au module microprocesseur peuvent être modifiées ou remplacées par des données transmises par l'unité extérieure (48) par l'intermédiaire du dispositif télémétrique (46).

27. Système selon l'une des revendications 21 à 26, **caractérisé en ce qu'**il est prévu au moins deux zones de mémoire (S₁, S₂) servant à enregistrer et à restituer au moins le programme d'exploitation de l'organe de traitement de signal (42).

28. Système selon l'une des revendications 22 à 27, **caractérisé en ce que** le dispositif formant mémoire intermédiaire comporte au moins deux zones de mémoire (S₄, S₅) servant à enregistrer et à restituer des données transmises par l'unité extérieure (48) par l'intermédiaire du dispositif télémétrique (46).

29. Système selon l'une des revendications 11 à 28, **caractérisé en ce que**, de plus, une zone de mémoire à lecture seule (S₀) préprogrammée et ne pouvant pas être écrasée par surfrappe est associée à l'organe de traitement de signal (42).

30. Système selon l'une des revendications 11 à 29, **caractérisé en ce que** le dispositif télémétrique (46) est également conçu pour transmettre des paramètres de fonctionnement entre la partie implantable (34) du système et l'unité extérieure (48).

31. Système selon l'une des revendications précédentes, **caractérisé en ce que** ledit système est complètement implantable et est muni d'au moins un capteur acoustique implantable (20), **en ce que** l'unité d'alimentation en énergie électrique comporte, côté implant, un élément accumulateur électrique rechargeable (25) et **en ce qu'**il est prévu un dispositif de charge sans fil transcutané (22, 23) servant à charger l'élément accumulateur.

32. Système selon la revendication 31, **caractérisé par** une télécommande sans fil (21) permettant au porteur d'implant de commander les fonctions d'implant.

33. Système selon l'une des revendications 1 à 30, **caractérisé en ce que** ledit système est partiellement implantable, auquel cas au moins un capteur acoustique (20), l'unité électronique de traitement de signal (62), l'unité d'alimentation en énergie (52) ainsi qu'une unité de modulation-émission (63) sont contenus dans un module extérieur (64) destiné à être porté extérieurement sur le corps, de préférence sur la tête, au-dessus de l'implant (34'), et **en ce que** l'implant est passif au plan énergétique et reçoit son énergie de fonctionnement et des données de commande de transducteur par l'intermédiaire de l'unité de modulation-émission placée dans le module extérieur.

34. Système selon l'une des revendications précédentes, **caractérisé en ce que** ledit système est conçu sous forme de système biaural servant à corriger un trouble auditif des deux oreilles, lequel système comporte deux unités de système qui sont chacune associées à l'une des deux oreilles.

35. Système selon la revendication 34, **caractérisé en ce que** les deux unités de système sont sensiblement identiques l'une à l'autre.

36. Système selon la revendication 34, **caractérisé en ce que** l'une des unités de système est conçue comme unité maîtresse et l'autre unité de système comme unité esclave commandée par l'unité maîtresse.

37. Système selon l'une des revendications 34 à 36, **caractérisé par** une liaison filaire implantable (52) par l'intermédiaire de laquelle les modules de traitement de signal (34) communiquent ensemble de manière à obtenir dans les deux unités de système une optimisation du traitement de signal biaural et de la commande de l'ensemble transducteur-groupement.

38. Système selon l'une des revendications 34 à 36, **caractérisé par** une liaison sans fil (53), de préférence une section haute fréquence bidirectionnelle, par l'intermédiaire de laquelle les modules de traitement de signal (34) communiquent ensemble de manière à obtenir dans les deux unités de système une optimisation du traitement de signal biaural et de la commande de l'ensemble transducteur-groupement.

39. Système selon l'une des revendications 34 à 36, **caractérisé en ce que**, moyennant l'utilisation de coupleurs ultrasoniques (55), les modules de traitement de signal (34) communiquent ensemble par l'intermédiaire d'une section à ultrasons (54) à couplage par conduction osseuse de manière à obtenir dans les deux unités de système une optimisation du traitement de signal biaural et de la commande de l'ensemble transducteur-groupement.

40. Système selon l'une des revendications 34 à 36, **caractérisé en ce qu'**aux modules de traitement de signal (34) sont associées des électrodes implantables (56) qui, à l'état implanté, font partie d'une section de transmission de données (57) à conduction par le tissu cellulaire du porteur d'implant et qui permettent aux modules de traitement de signal (34) des deux unités de système de communiquer.
